# EUROPEAN PATENT APPLICATION

(11) **EP 4 553 169 A1**
(43) Date of publication of application: **14.05.2025**
(21) Application number: 23383143.7
(22) Date of filing: 08.11.2023
(51) Int. Cl.: C12Q 1/6816, C12Q 1/6853

(54) **METHODS AND REAGENTS FOR THE MULTIPLEXED DETECTION OF NUCLEIC ACIDS**

(71) Applicant: Paperdrop Diagnostics S.L., 08301 Mataró, Barcelona (ES)
(72) Inventor: RUBIO MONTERDE, Ana, E-08195 Sant Cugat del Vallès (ES); GALLEGOS MAMOLEJO, Marc, E-08173 Sant Cugat del Vallès (ES); RIVAS TORCATES, Lourdes, E-08202 Sabadell (ES); RUIZ GATELL, Maria, E-08224 Terrassa (ES)
(74) Representative: ABG Intellectual Property Law, S.L.

(57) **Abstract**

The present invention relates to a method for the multiplexed detection of nucleic acids in a sample. This is accomplished by using novel sets of primer pairs containing tails which can be selectively captured by complementary tail sequences with negligible cross-hybridization. The invention relates also to reagents, kits and devices used to implement the multiplexed detection method.

## Description

### FIELD OF THE INVENTION

The invention relates to the field of nucleic acid detection and, more in particular, to methods and reagents for the multiplexed detection of nucleic acids in a sample.

### BACKGROUND ART

Nucleic acid detection has become a central to a wide variety of scientific techniques and underpins various detection methods, such as detection of pathogens, genetic conditions and gene expression levels. Nucleic acids detection is becoming increasingly important for diagnostic and therapeutic use. Traditional methods for nucleic acid detection typically require amplification of target nucleic acid prior to detection. In order to have a correct interpretation of the levels of the detected sequences, different systems have been developed to use specific set of primers to amplify nucleic acid sequences and then capture the amplicons and identify the presence of the nucleic acid sequences.

The identification of a complex sample with more than one target nucleic acid sequence requires the presence of more than one target specific primer pairs and a capture system that is integrated in the primer pair. Most of these capture systems are based on different labels and consequently require reagents capable of specifically recognizing each label. This detection of several target sequences could generate problems of cross hybridization of the tagged amplicon and the complementary capture elements because of deficiencies in the design or lack of specificity of the capture system being unable to join to the correct amplicon. This results in a lack of consistency of the results as it is not possible to differentiate the results from the different target sequences.

Thus, there is still a need for the identification of alternative methods for the multiplexed detection of nucleic acids that avoid the need to find different labels and the consequent capture antibodies and to avoid the problem of cross-reactivity that generate inaccurate results.

### SUMMARY OF THE INVENTION

Described herein is a set of tailed primers pairs that allows the detection of multiple different sequences by hybridization by using complementary nucleic acids. These primers can be used for the multiplexed detection of a plurality of targets by using oligonucleotides which comprise a first region having the sequence of tail and second region having a sequence specific for the target.

Thus, in a first aspect the present invention relates to a set of primer pairs for the multiplexed detection of a series of different target nucleic acids wherein the set contains at least two types of primer pairs being each type of primer pair specific for a different target nucleic acid wherein, for each given type of primer pair:
(i) The forward primer contains a target-specific region which is capable of specifically hybridizing to a target nucleic acid within the series of different target nucleic acids and a barcode region which comprises a sequence selected from the group consisting of SEQ ID NO: 1 to 8 or a variant thereof capable of specifically hybridizing to a nucleic acid having a sequence which is complementary to SEQ ID NO:1 to 8 and
(ii) the reverse primer contains a target-specific region which is capable of specifically hybridizing to said target nucleic acid and which is labelled in its 5' end with a detectable tag.
wherein the barcode region within the forward primer in a type of primer pairs is different from the barcode regions within the forward primers in the other types of primer pairs.

In a second aspect, the invention provides a method for the multiplexed amplification of a series of target nucleic acids in a sample comprising contacting the sample with a set of primer pairs as defined in the first aspect wherein the set of primers contains primer pairs containing forward and reverse primers specific for each of the target nucleic acids in the sample, wherein said contacting is carried out under conditions adequate for the amplification of the target nucleic acid thereby generating amplicons from each target nucleic acid, wherein each amplicon contains a tail comprising the sequence of the barcode region forming part of the specific forward primer and the detectable tag forming part of the specific reverse primer.

In a third aspect, the present invention relates to a method for the multiplexed detection of a series of target nucleic acids in a sample comprising the steps of:
(i) Generating amplicons from each type of target nucleic acid in a sample by means of a method as defined in the second aspect,
(ii) Contacting the amplicons formed in step (i) with a support, said support containing different types of capture probes immobilized each of them at spatially predefined positions within the support, wherein each type of capture probe contains a region which contains a sequence complementary to a type of barcode region present in the amplicons, said contacting being carried out under conditions adequate for the hybridization between the tail in each type of amplicon and the complementary barcode region in the capture probes
wherein detection of the detectable label at one or more of the spatially predefined positions is indicative that the sample contains the target nucleic acid which forms part of the tailed amplicon containing a sequence which is complementary to the sequence of the capture probe.

A set of probes comprising different types of probes, wherein each type of probe contains a region having a sequence substantially complementary to barcode region wherein the barcode regions are as defined in any of SEQ ID NO:1 to 8 or a variant thereof capable of specifically hybridizing to a nucleic acid having a sequence which are complementary to SEQ ID NO.1 to 8 wherein the region having a sequence complementary to a type of barcode region in each type of capture probe is different from that in the other capture probes of the set.

In a fifth aspect, the invention relates to a Method for the capture of a plurality of different types of nucleic acid present in a sample wherein each type of nucleic acid within the sample contains a barcode sequence as defined in any of SEQ ID NO:1 to 8 or a variant thereof capable of specifically hybridizing to a nucleic acid having the sequence of SEQ ID NO: 1 to 8 and wherein the barcode region in each type of nucleic acid is different from the barcode regions in the other types of nucleic acids in the sample, the method comprising contacting the sample with a set of probes as defined in any of claims 27 or 28, said contacting being carried out under conditions adequate for the hybridization between the barcode regions present in each type of nucleic acid and the sequences complementary to said barcode region in each type of capture probes.

In a sixth aspect, the invention relates to a set of primers wherein the set of primers contains at least two types of primers being each type of primer specific for a different target nucleic acid wherein, for each given type of primer, it contains a target-specific region which is capable of specifically hybridizing to the target nucleic acid within the series of different target nucleic acids and a barcode region which comprises a sequence selected from the group consisting of SEQ ID NO: 1 to 8, wherein the barcode region within the primer in a type of primer pairs is different from the barcode regions within the primers in the other types of primers.

In a seventh aspect, the invention relates to a kit comprising a set of primer pairs as defined in the first aspect and a set of capture probes as defined in the fourth aspect.

In an eighth aspect, the invention relates to a use of a kit according to the seventh aspect in a method for the multiplexed detection of a series of target nucleic acids in a sample wherein the capture probes are immobilized in a support at spatially predefined locations.

### DESCRIPTION OF THE FIGURES

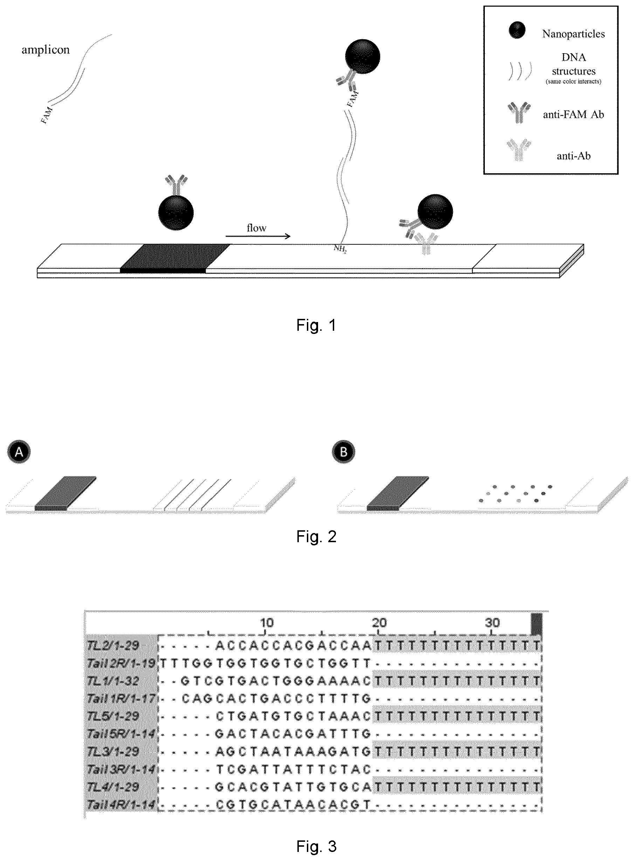

**Figure 1****:** Nanoparticles-based LF design
**Figure 1****:** Multiplexed models of mLFTs: A) multiple lines, B) dots array
**Figure 3****:** Sequences of the capture probes aligned with their corresponding complementary tails in order to hybridize and capture the amplicon.
**Figure 4****:** Upper row) Agarose gels showing the amplicons obtained by amplification using forward primers with tails. Lower row) Detection in lateral flow strips of the amplicons using the capture probes as TLs.
**Figure 5****:** LF strips of multiple lines for detection of the amplification products in quadruplex.
**Figure 6****:** Sequences of the 8 capture probes aligned together with the complementary tails.
**Figure 7****:** Example of a design of the array for detection of antibiotic resistance genes.
**Figure 8****:** Structure of Spacers 9 and C3
**Figure 9****:** Agarose gels where can be observed the amplification products obtained using a forward primer labelled with tails with iSpC3 o iSp9.
**Figure 10****:** Agarose gel where can be observed the amplification products obtained using primers without tails, reverse labelled with tail and forward labelled with tail.
**Figure 11:** A) Positive for all targets. B) Positive for OXA-48 (TL1), IPC (TL2), CTX-M-1 (TL3) and VIM (TL5). C) Positive for NDM (TL6), OXA-23 (TL7), KPC (TL8) and IMP (TL9). D) Negative for all targets.
**Figure 12:** A) Positive for IPC (TL2). B) Positive for OXA-48 (TL1). C) Positive for CTX-M-1 (TL3). D) Positive for VIM (TL5).
**Figure 13:** A) Positive for NDM (TL6). B) Positive for OXA-23 (TL7). C) Positive for KPC (TL8). D) Positive for IMP (TL9).
**Figure 14****:** Detection of the amplified product of SARS-CoV-2 by TL1.
**Figure 15****:** Detection in duplex using TL1 and TL2 of amplified products of SARS-CoV-2.

### DETAILED DESCRIPTION OF THE INVENTION

The authors of the present invention have designed a series of barcode sequences that, when incorporated in nucleic acids, allow the selective capture of the nucleic acids using sequences complementary to the barcode sequences. Since the recognition of the barcode sequences by the complementary sequence is highly specific and devoid of any cross-hybridization, the barcode sequences are particularly useful when used in multiplexed detection assays. Accordingly, the invention is defined by the following aspects:

### Set of primer pairs for the multiplexed detection

In a first aspect the present invention relates to a set of primer pairs for the multiplexed detection of a series of different target nucleic acids wherein the set contains at least two types of primer pairs being each type of primer pair specific for a different target nucleic acid wherein, for each given type of primer pair:
(i) The forward primer contains a target-specific region which is capable of specifically hybridizing to a target nucleic acid within the series of different target nucleic acids and a barcode region which comprises a sequence selected from the group consisting of SEQ ID NO: 1 to 8. The forward primer contains a target-specific region which is capable of specifically hybridizing to a target nucleic acid within the series of different target nucleic acids and a barcode region which comprises a sequence selected from the group consisting of SEQ ID NO: 1 to 8 or a variant thereof capable of specifically hybridizing to a nucleic acid having a sequence which are complementary to SEQ ID NO.1 to 8 and,
(ii) the reverse primer contains a target-specific region which is capable of specifically hybridizing to said target nucleic acid and which is labelled in its 5' end with a detectable tag
wherein the barcode region within the forward primer in a type of primer pairs is different from the barcode regions within the forward primers in the other types of primer pairs.

The term "set", when used to define the primer pairs, refers to a plurality of types of primer pairs, each of them characterized in that each type of primer pair contains a barcode sequence that is different from the barcode sequence present in the other types of primer pairs within the set. In some embodiment, the set of primer pairs contains 2 types of primer pairs, 3 types of primer pairs, 4 types of primer pairs, 5 types of primer pairs, 6 types of primer pairs, 7 types of primer pairs or 8 types of primer pairs.

As it is used herein, the term "primer" refers to a short strand of nucleic acid that is complementary to a sequence in another nucleic acid and serves as a starting point for DNA synthesis. A primer is selected to be "substantially" complementary to a strand of specific sequence of the template. A primer must be sufficiently complementary to hybridize with a template strand for primer elongation to occur. A primer sequence need not reflect the exact sequence of the template. For example, a non-complementary nucleotide fragment may be attached to the 5' end of the primer, with the remainder of the primer sequence being substantially complementary to the strand. Non-complementary bases or longer sequences can be interspersed into the primer, provided that the primer sequence has sufficient complementarity with the sequence of the template to hybridize and thereby form a template primer complex for synthesis of the extension product of the primer.

The term "nucleic acid", "nucleotide sequence" or "polynucleotide" is used interchangeably in the present invention to refer to the polymeric form of the ribonucleoside phosphate ester (adenosine, guanosine, uridine or cytidine; "RNA molecules") or deoxy ribonucleosides (deoxiadenosine, deoxyguanosine, deoxythymidine or deoxycytidine; "DNA molecules") or any phosphoester analog thereof such as phosphorothioates and thioesters, in a single stranded or double stranded form. Thus, the term includes single stranded DNA or RNA molecules. It also includes double stranded molecules formed by DNA-DNA, DNA-RNA and RNA-RNA strands. The term "nucleic acid sequence" and, in particular, the DNA or RNA molecule, refers only to the primary or secondary structure of the molecule and does not limit any particular type of tertiary structure. Thus, this term encompasses double stranded DNA, as comprised in linear or circular DNA molecules, supercoiled DNA plasmids and chromosomes. An "oligonucleotide," as it is used herein, is a short polynucleotide or a portion of a polynucleotide. An oligonucleotide typically contains a sequence of about two to about one hundred bases. The word "oligo" is sometimes used in place of the word "oligonucleotide".

In general, the term "target nucleic acid" refers to a nucleic acid molecule or polynucleotide in a starting population of nucleic acid molecules having a target sequence whose presence, amount, and/or nucleotide sequence, or changes in one or more of these, are desired to be determined. In general, the term "target sequence" refers to a nucleic acid sequence on a single strand of nucleic acid. The target sequence may be a portion of a gene, a regulatory sequence, genomic DNA, cDNA, RNA including mRNA, miRNA, rRNA, or others. The target sequence may be a target sequence from a sample or a secondary target such as a product of an amplification reaction. A target polynucleotide is not necessarily any single molecule or sequence. For example, a target polynucleotide may be any one of a plurality of target polynucleotides in a reaction, or all polynucleotides in a given reaction, depending on the reaction conditions. For example, in a nucleic acid amplification reaction with random primers, all polynucleotides in a reaction may be amplified. As a further example, a collection of targets may be simultaneously assayed using polynucleotide primers directed to a plurality of targets in a single reaction. As yet another example, all or a subset of polynucleotides in a sample may be modified by the addition of a primer-binding sequence (such as by the ligation of adapters containing the primer binding sequence), rendering each modified polynucleotide a target polynucleotide in a reaction with the corresponding primer polynucleotide(s).

The expression "specifically", when referred to the hybridization of a target DNA sequence by another specific DNA sequence, means that the primers are capable of hybridizing to the target DNA sequence but they are not capable of hybridizing other sequences different from the target DNA sequence, thus allowing a specific detection of said sequence.

The term "target-specific region", as it is used herein, refers to a region within the forward primer which is capable to specifically hybridizing to a target nucleic acid with the series of different target nucleic acids and a barcode region. In a particular embodiment, the target-specific region within the forward primer is located 3' with respect to the barcode region.

As it is used herein, the term "barcode region", refers to a forward primer region which comprises a sequence selected from the group consisting of SEQ ID NO: 1 to 8 as defined below

**Table 1: Sequences of the barcode regions**

| **Name** | **Sequences** |
|---|---|
| SEQ ID NO: 1 | GTT TTC CCA GTC AC |
| SEQ ID NO: 2 | TTG GTC GTG GTG GT |
| SEQ ID NO: 3 | CAT CTT TAT TAG CT |
| SEQ ID NO: 4 | GTT TAG CAC ATC AG |
| SEQ ID NO: 5 | AAC GCT CAC CCA TG |
| SEQ ID NO: 6 | CGG AGT TGA GGT GA |
| SEQ ID NO: 7 | TCT AGT AGT GGA TC |
| SEQ ID NO: 8 | CTA TGG TGA ACG AA |

In some embodiments:
- the barcode region contains a sequence which is a variant of SEQ ID NO:1 and which is capable of specifically hybridizing to a nucleic acid having a sequence which is complementary to SEQ ID NO:1.
- the barcode region contains a sequence which is a variant of SEQ ID NO:2 and which is capable of specifically hybridizing to a nucleic acid having a sequence which is complementary to SEQ ID NO:2.
- the barcode region contains a sequence which is a variant of SEQ ID NO:3 and which is capable of specifically hybridizing to a nucleic acid having a sequence which is complementary to SEQ ID NO:3.
- the barcode region contains a sequence which is a variant of SEQ ID NO:4 and which is capable of specifically hybridizing to a nucleic acid having a sequence which is complementary to SEQ ID NO:4.
- the barcode region contains a sequence which is a variant of SEQ ID NO:5 and which is capable of specifically hybridizing to a nucleic acid having a sequence which is complementary to SEQ ID NO:5.
- the barcode region contains a sequence which is a variant of SEQ ID NO:6 and which is capable of specifically hybridizing to a nucleic acid having a sequence which is complementary to SEQ ID NO:6.
- the barcode region contains a sequence which is a variant of SEQ ID NO:7 and which is capable of specifically hybridizing to a nucleic acid having a sequence which is complementary to SEQ ID NO:7 and/or
- the barcode region contains a sequence which is a variant of SEQ ID NO:8 and which is capable of specifically hybridizing to a nucleic acid having a sequence which is complementary to SEQ ID NO:8.

As used herein, "specifically hybridizes" refers to preferential hybridization under hybridization conditions where two nucleic acids, or portions thereof, that are substantially complementary, hybridize to each other and not to other nucleic acids that are not substantially complementary to either of the two nucleic acids. For example, specific hybridization includes the hybridization of a barcode region to a portion of a capture nucleic acid that is substantially complementary to the barcode or capture nucleic acid. In some embodiments nucleic acids, or portions thereof, that are configured to specifically hybridize are often about 80 percent or more, 81 percent or more, 82 percent or more, 83 percent or more, 84 percent or more, 85 percent or more, 86 percent or more, 87 percent or more, 88 percent or more, 89 percent or more, 90 percent or more, 91 percent or more, 92 percent or more, 93 percent or more, 94 percent or more, 95 percent or more, 96 percent or more, 97 percent or more, 98 percent or more, 99 percent or more or 100 percent complementary to each other over a contiguous portion of nucleic acid sequence. A specific hybridization discriminates over non-specific hybridization interactions (e.g., two nucleic acids that are not configured to specifically hybridize, e.g., two nucleic acids that are 80 percent or less, 70 percent or less, 60 percent or less or 50 percent or less complementary) by about 2-fold or more, often about 10-fold or more, and sometimes about 100-fold or more, 1000-fold or more, 10,000- fold or more, 100,000-fold or more, or 1 ,000,000-fold or more. Two nucleic acid strands that are hybridized to each other can form a duplex which includes a double stranded portion of nucleic acid.

A nucleic acid, or a portion thereof, "hybridizes" to another nucleic acid under conditions such that non-specific hybridization is minimal at a defined temperature in a physiological buffer (e.g., pH 6-9, 25-150 mM chloride salt). In some cases, a nucleic acid, or portion thereof, hybridizes to a conserved sequence shared among a group of target nucleic acids. In some cases, the barcode region can hybridize to a sequence being complementary to any of SEQ ID NO:1 to 8 if there are at least about 6, 8, 10, 12, 14, 16, or 18 contiguous complementary nucleotides of the sequences defined in SEQ ID NO:1 to 8 including "universal" nucleotides that are complementary to more than one nucleotide partner. Alternatively, the barcode region can hybridize to a sequence being complementary to any of SEQ ID NO:1 to 8 if there are 0, or fewer than 2 or 3 complementarity mismatches over at least about 10, 12 or 14 contiguous nucleotides. In some embodiments, the defined temperature at which specific hybridization occurs is room temperature. In some embodiments, the defined temperature at which specific hybridization occurs is higher than room temperature. In some embodiments, the defined temperature at which specific hybridization occurs is at least about 37, 40, 42, 45, 50, 55, 60, 65, 70, 75, or 80 degrees centigrade. In some embodiments, the defined temperature at which specific hybridization occurs is 37, 40, 42, 45, 50, 55, 60, 65, 70, 75, or 80 degrees centigrade.

In some embodiments, the variants of the barcode regions have one mismatch with respect to the sequences of SEQ ID NO:1 to 8. In some embodiments, the variants of the barcode regions have two mismatches with respect to the sequences of SEQ ID NO:1 to 8. In some embodiments, the variants of the barcode regions have three mismatches with respect to the sequences of SEQ ID NO:1 to 8. In some embodiments, the variants of the barcode regions have four mismatches with respect to the sequences of SEQ ID NO:1 to 8. The mismatches in the variants may be contiguous or scattered throughout the barcode region.

In another particular embodiment, the target-specific region and the barcode region within the forward primer from each type of primer pair are connected by a spacer and/or wherein the target-specific region and the detectable tag in the reverse primer from each type of primer pair are connected by a spacer group. This spacer is referred hereinafter as "primer spacer".

While the sequences defined in Table 1 consist each of 14 nucleotides, the barcode regions may be longer provided that the barcode sequences are located within the region. In a particular embodiment, the length of the barcode region in the forward primers within each type of primer pair is of between 14 and 20 nucleotides. More preferably, the length of the barcode region in the forward primers within each type of primer pair is of 14 nucleotides, 16 nucleotides or 18 nucleotides.

The term "primer spacer", as it is used herein, refers to a molecule that links the target-specific region to either the barcode region within the forward primer or the detectable tag within the reverse primer. The primer spacer acts as a hinge region, providing space between both elements. In a preferred embodiment the primer spacer contains a is a polyethyleneglycol group or an alkyl group. In a more preferred embodiment the primer spacer group has the following structure: 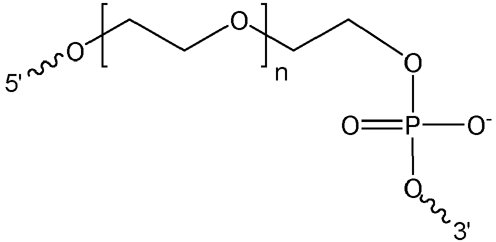 wherein n is from 0 to 18. In a more preferred embodiment, the primer spacer has the structure:

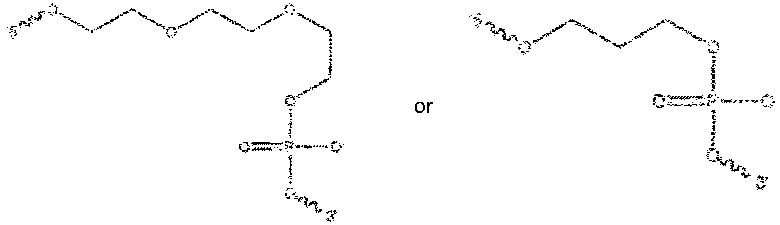

In a more preferred embodiment, the primer spacer is selected from a group consisting of: hjexaethylene glycosl, tetraethylenglycoi or triethylene glycol.

The reverse primer contains a target-specific region which is capable of specifically hybridizing to said target nucleic acid and which is labelled in its 5' end with a detectable tag.

As it is used herein, the term "detectable tag", refers to an atom or a molecule used to specifically detect a molecule or substance with a label, from among the same type of molecules or substances without a label. The term "detectable tag" can include any composition detectable by spectroscopic, photochemical, biochemical, immunochemical, electrical, optical, or chemical means. Example detectable labels include, but are not limited to, fluorescent nanoparticles (e.g., quantum dots (Qdots)), metal nanoparticles(e.g., gold nanoparticles), metal-oxide nanoparticles, fluorescent dyes (e.g., fluorescein, texas red, rhodamine, green fluorescent protein, and the like, see, e.g., Molecular Probes, Eugene, Oreg., USA), radiolabels (e.g., ³H, ¹²⁵I, ³⁵S, ¹⁴C, ³²P, ⁹⁹TC, ²⁰³Pb, ⁶⁷Ga, ⁶⁸Ga, ⁷²As, ¹¹¹In, ^{113m}In, ⁹⁷Ru, ⁶²Cu, ⁵²Fe, ^{52m}Mn, ⁵¹cr, ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²pr, ¹⁴³pr, ¹⁹⁸Au, ¹⁹⁹Au, ¹⁶¹Tb, ¹⁰⁹pd, ¹⁶⁵Dy, ¹⁴⁹pm, ¹⁵¹pm ¹⁵³Sm, ¹⁵⁷Gd, ¹⁵⁹Gd, ¹⁶⁶Ho, ¹⁷²TM, ¹⁶⁹Y b, ¹⁷⁵Yb, ¹⁷⁷Lu, ¹⁰⁵Rh, ¹¹¹Ag, and the like), enzymes (e.g., horse radish peroxidase, alkaline phosphatase and others commonly used in an ELISA), various colorimetric labels, magnetic or paramagnetic labels (e.g., magnetic and/or paramagnetic nanoparticles), spin labels, radio-opaque labels, and the like. In some embodiments, the detectable tag is a fluorophore, a radioisotope, a colorimetric substrate or enzymes or a heterologous epitope for which specific antibodies are commercially available. In a particular embodiment, the detectable tag within the reverse primer in each primer pair is connected to the 5' end of the reverse primer. In a preferred embodiment, the detectable tag is selected from a group consisting of 6-carboxyfluorescein (FAM), biotin, digoxigenin, fluorescein or an oligonucleotide having a predefined sequence.

The detectable tag includes both tags which are directly detected using any of the spectroscopic, photochemical, biochemical, immunochemical, electrical, optical, or chemical means defined above. In other embodiments, the detectable tag is detected indirectly by using a ligand which is capable of specifically binding the detectable tag and which is coupled to a nanoparticle and wherein the tag is detected by its association to the nanoparticle. Suitable nanoparticles to be used in the indirect detection of the tags will be explained in detail below in the context of the second method of the invention.

The term "comprising" or "comprises", as it is used herein, discloses also "consisting of" according to the generally accepted patent practice.

### Method for multiplexed amplification

In a second aspect, the invention relates to a method, hereinafter "first method of the invention", for the multiplexed amplification of a series of target nucleic acids in a sample comprising contacting the sample with a set of primer pair as defined in the first aspect wherein the set of primers contains primer pairs containing forward and reverse primers specific for each of the target nucleic acids in the sample, wherein said contacting is carried out under conditions adequate for the amplification of the target nucleic acid thereby generating amplicons from each target nucleic acid, wherein each amplicon contains a tail comprising the sequence of the barcode region forming part of the specific forward primer and the detectable tag forming part of the specific reverse primer.

As used herein, a multiplexed amplification is an amplification reaction that detects multiple distinct target sequences per reaction.

As it is used herein, the term "amplification" refers to any known procedure for obtaining multiple copies of a target nucleic acid sequence or its complement or fragments thereof. The multiple copies may be referred to as amplicons or amplification products. Amplification of "fragments" refers to production of an amplified nucleic acid that contains less than the complete target nucleic acid or its complement, e.g., produced by using an amplification oligonucleotide that hybridizes to, and initiates polymerization from, an internal position of the target nucleic acid. Known amplification methods include both thermal and isothermal amplification methods. Replicase-mediated amplification, polymerase chain reaction (PCR), ligase chain reaction (LCR), strand-displacement amplification (SDA), and transcription-mediated or transcription-associated amplification are non-limiting examples of nucleic acid amplification methods. In a particular embodiment, the amplification is an isothermal amplification. As used herein, "under isothermal conditions" means that the amplification reaction is conducted at a relatively constant temperature, thereby obviating the need for a thermal cycler. Exemplary, isothermal amplifications include, but is not limited to, Recombinase Polymerase Amplification (RPA), Loop Mediated Isothermal Amplification (LAMP), Helicasedependent isothermal DNA amplification (HDA), Rolling Circle Amplification (RCA), Nucleic acid sequence-based amplification (NASBA), strand displacement amplification (SDA), nicking enzyme amplification reaction (NEAR), Polymerase Spiral Reaction (PSR), Hybridization Chain Reaction (HCR), Primer Exchange Reaction (PER), Signal Amplification by Exchange Reaction (SABER), transcription-based amplification system (TAS), Self-sustained sequence replication reaction (3 SR), Single primer isothermal amplification (SPIA), and cross-priming amplification (CPA).

In general, the amplification reaction is performed in a reaction mixture. By "reaction mixture," as used herein, is meant a composition including the relevant components to allow an amplification reaction to be performed. An exemplary reaction mixture can include, without limitation, a nucleic acid sample, primer pairs, and a suitable enzyme, such as a polymerase. A "polymerase" refers to an enzyme that performs template-directed synthesis of polynucleotides, e.g., DNA and/or RNA. The term encompasses both the full-length polypeptide and a domain that has polymerase activity. DNA polymerases are well-known to those skilled in the art, including but not limited to DNA polymerases isolated or derived from *Pyrococcus furiosus*, *Thermococcus litoralis*, and *Thermotoga maritime*, or modified versions thereof. Additional examples of commercially available polymerase enzymes include, but are not limited to: Klenow fragment (New England Biolabs(R) Inc), Taq DNA polymerase (QIAGEN), 9 degrees N^{™} DNA polymerase (New England Biolabs(R) Inc.), Deep Vent^{™} DNA polymerase (New England Biolabs(R) Inc.), Manta DNA polymerase (Enzymatics(R)), Bst DNA polymerase (New England Biolabs(R) Inc.), and phi29 DNA polymerase (New England Biolabs(R) Inc.).

One of skill in the art will appreciate that the reaction mixture may also include other components such as buffers, stabilisers, templates, nucleotides and the like and that these components may be dictated by the amplification reaction being performed.

It is to be understood that amplification parameters, such as nucleotide concentration, nucleic acid polymerases used for the amplification, buffer composition, number of amplification cycles, temperatures during the cycles, can be optimized as described herein or known in the art.

The term "a series of target nucleic acids", as it is used herein, refers to the different types of nucleic acids that can be amplified in a multiplexed setting according to the first method of the invention and that consists of least 2, at least 3, at least 4, at least 5, at least 6, at least 7 or at least different target nucleic acids. In some embodiments, each type of primer pair binds to a different target polynucleotide. In other embodiment, two or more of the primer pairs bind to different regions from the same polynucleotide. Target nucleic acids include, without limitation, RNA or DNA, for example, chromosomal DNA, mitochondrial DNA, messenger RNA, ribosomal RNA, transfer RNA, viral RNA and extrachromosomal DNA, such as virulence plasmids.

The term "sample", as it is used herein, relates to a material or mixture of materials containing one or more components of interest. The term "sample" includes "biological sample" which refers to a sample obtained from a biological subject, including a sample of biological tissue or fluid origin, obtained, reached, or collected *in vivo* or *in situ.* Suitable biological samples include a forensic sample, a synthetic sample or an environmental sample.

As it is used herein, the term "conditions adequate for the amplification" means those conditions known by the skilled person by which a nucleic acid can be amplified. Those skilled in the art will be able to determine adequate conditions to perform the multiplex amplification step, such as the optimum primer length, temperature and specificity.

The term "amplicon", as it is used herein, refers to an amplification product such as a nucleic acid that is amplified by a PCR reaction or other amplification reaction or method. The amplicons may be of any size, such as, for example, between about 50 and about 100 bp, between about 100 bp and about 200 bp, or between about 200 bp and about 1kb, or between about 500 bp and about 5000 bp, or between about 2000 and about 20000 bp. The first method according to the invention allows the generation of one type of amplicon for each of the types of primer pairs used in the reaction. In some aspects, the first method according to the present invention results in the generation of two type of amplicons, three types of amplicons, four types of amplicons, five types of amplicons, six types of amplicons, seven types of amplicons or eight types of amplicons.

All the terms and embodiments described in the first aspect of the invention are equally applicable to this aspect of the invention.

### Method for multiplexed detection

In a third aspect, the present invention relates to a method, hereinafter "second method of the invention", for the multiplexed detection of a series of target nucleic acids in a sample comprising the steps of:
(i) Generating amplicons from each type of target nucleic acid in a sample by means of a method as defined in the second aspect,
(ii) Contacting the amplicons formed in step (i) with a support, said support containing different types of capture probes immobilized each of them at spatially predefined positions within the support, wherein each type of capture probe contains a region which contains a sequence complementary to a type of barcode region present in the amplicons, said contacting being carried out under conditions adequate for the hybridization between the tail in each type of amplicon and the complementary barcode region in the capture probes
wherein detection of the detectable label at one or more of the spatially predefined positions is indicative that the sample contains the target nucleic acid which forms part of the tailed amplicon containing a sequence which is complementary to the sequence of the capture probe.

In a first step, the second method of the invention, comprises, or consists of, generating amplicons from each type of target nucleic acid in a sample by means of a method as defined in the first method, corresponding to the second aspect of the invention. Both concepts "amplicon" and "target nucleic acid" have been previously defined in relation to the first and second aspect of the invention and are incorporated herein by reference.

In a second step, the second method of the invention, comprises, or consists of, contacting the amplicons formed in first step with a support, said support containing different types of capture probes immobilized each of them at spatially predefined positions within the support and wherein the contacting is carried out under conditions adequate for the hybridization between the tail in each type of amplicon and the complementary barcode region in the capture probes.

The term "capture probe" or "probe" refers to a probe that is immobilised on the surface of a substrate, it recognises specifically a target in a sample and it binds specifically to and immobilises that target to the surface of the substrate.

In some embodiments, said contacting of the amplicons with the capture probes is performed after isolation or purification of the amplicons from the amplification of the target nucleic acids. In other words, the method further comprises a step of isolating or purifying the amplicon from the amplification reaction prior to contacting with the probe.

In order to ensure effective hybridization of the capture probes to the target amplicons, a spacer can be introduced between the surface and the hybridizing sequence. This region, hereinafter referred to as "capture probe spacer" can be composed of units that are capable of hybridization such as nucleotides and nucleotide analogs. Alternatively, the capture probe spacer can be composed of units that are not capable of hybridization such as a hydrocarbon chain. The hydrocarbon chain can also contain atoms taken from the 5^{th} or 6^{th} group of the periodic system, preferably nitrogen, phosphorous, oxygen or sulfur. The capture probe spacer can also be composed of both nucleotide and non-nucleotide units.

In one particularly preferred embodiment the capture probes are covalently immobilized on the surface of the detection zone via their 3'- or 5'-termini without a capture probe spacer on a planar substrate that is coated with a polymeric layer. In this embodiment, the polymeric layer ensures effective hybridization of the capture probes to the target. In another particularly preferred embodiment, the capture probes are immobilized on a planar surface via their 3'- or 5 '-termini with a short capture probe spacer containing 2-20 units capable of hybridization, particularly preferred 2-10 units capable of hybridization on the detection zone. A variant in which the capture probes are covalently immobilized on the detection zone via their 3'- or 5'-termini with a capture probe spacer containing 3-30 ethylene glycol units is particularly preferred. Examples for spacer molecules that can be attached to the 5'-terminus of capture probes include the commercially available Spacer Phosphoramidite 9 (3 ethylene glycol units and one phosphate unit), 18 (6 ethylene glycol units and one phosphate unit) and C3 (propanol unit and phosphate unit) as well as the Spacer Cl 2 CE Phosphoramidite (dodecanol unit and phosphate unit) from Glen Research, Sterling, Virginia, USA. One example for a spacer molecule that can be attached to the 3'-terminus of the capture probes is the 3' Spacer 9 CPG (3 ethylene glycol units) which is also commercially available from Glen Research, Sterling, Virginia, USA.

In a particular embodiment, the capture probe contains 16 nucleotides of which 14 of them are complementary to the barcode region. In a preferred embodiment the capture probe spacer is an oligoT, oligoC, oligoG, oligoU or oligoA sequence. Preferably, the spacer region is an oligoT or an oligoA sequence. More preferably, the oligoT or oligoA sequence contains about either 15 thymidine or 15 adenine nucleotides. In some embodiments, at least one of the capture probes comprises a polyA tail. In various embodiments, the polyA tail comprises at least 40, 45, 50, 55, 60, 65, 70, 75, 80, or more than 80 adenine nucleotides at the 5' or 3' end of the probe. In some embodiments, the polyA tail comprises a sequence comprising at least 65 nucleotides, wherein at least 80 percent of the nucleotides are adenine, and wherein the polyA sequence is interrupted by non-adenine nucleotides.

The term "conditions adequate for the hybridization", as it is used herein, means those conditions known by the skilled person by which a nucleic acid sequences can hybridize. Illustrative non-limitative examples of conditions adequate for the hybridization are present in the examples of the invention. Stable capture of nucleic acids of interest, e.g., while minimizing capture of extraneous nucleic acids can be achieved, for example, by balancing the number of target capture probes, the amount of overlap between the capture probes and the complementary barcode sequence and/or the stringency of the conditions under which the target capture probes, the nucleic acids, and the support capture probes are hybridized. Appropriate combinations of the number of target capture probes, the extent of the complementarity between the capture probes and the barcode sequence within the amplicons, and stringency of hybridization can, for example, be determined experimentally by one of skill in the art. For example, a particular number of capture probes and a particular set of hybridization conditions can be selected, while the number of nucleotides of complementarity between the capture probes and the barcode region is varied until hybridization of the different capture probes to a nucleic acid captures the nucleic acid while hybridization of a single target capture probe does not efficiently capture the nucleic acid. Similarly, the number of capture probes, the amount of complementarity, and stringency of hybridization can be selected such that the desired nucleic acid of interest is captured while other nucleic acids present in the sample are not efficiently captured. Stringency can be controlled, for example, by controlling the formamide concentration, chaotropic salt concentration, salt concentration, pH, organic solvent content, and/or hybridization temperature.

As noted, the Tm of any nucleic acid duplex can be directly measured, using techniques well known in the art. For example, a thermal denaturation curve can be obtained for the duplex, the midpoint of which corresponds to the Tm. It will be evident that such denaturation curves can be obtained under conditions having essentially any relevant pH, salt concentration, solvent content, and/or the like.

The Tm for a particular duplex (e.g., an approximate Tm) can also be calculated. For example, the Tm for an oligonucleotide-target duplex can be estimated using the following algorithm, which incorporates nearest neighbour thermodynamic parameters: Tm (Kelvin) = DELTAH degrees /(DELTAS degrees + R InCt), where the changes in standard enthalpy (DELTAH degrees) and entropy (DELTAS degrees) are calculated from nearest neighbor thermodynamic parameters. The calculated Tm is optionally corrected for salt concentration, e.g., Na+ concentration, using the formula 1/Tm(Na+) = 1/Tm(1M) + (4.29f (G.C)-3.95)x10-5 In[Na+] + 9.40x10-6 In2[Na+]. Other algorithms for calculating Tm are known in the art and are optionally applied to the present invention.

Each type of capture probe contains a region which contains a sequence complementary to a type of barcode region present in the amplicons, said contacting being carried out under conditions adequate for the hybridization between the tail in each type of amplicon and the complementary barcode region in the capture probes wherein detection of the detectable label at one or more of the spatially predefined positions is indicative that the sample contains the target nucleic acid which forms part of the tailed amplicon containing a sequence which is complementary to the sequence of the capture probe.

The capture probes are immobilized in spatially predefined locations within the support.

The spatially predefined regions may have defined locations in a regular array, which may correspond to a rectilinear pattern, circular pattern, hexagonal pattern, or the like. In embodiments, the pattern of regions includes concentric circles of regions, spiral patterns, rectilinear patterns, hexagonal patterns, and the like. In embodiments, the pattern of regions is arranged in a rectilinear or hexagonal pattern. A regular array of such regions is advantageous for detection and data analysis of signals collected from the arrays during an analysis. These spatially defined locations are separated by interstitial regions. As used herein, the term "interstitial region" refers to an area in a substrate or on a surface that separates other areas of the substrate or surface. The two regions that are separated from each other can be discrete, lacking contact with each other. In embodiments the interstitial region is continuous whereas the spatially defined locations are discrete, for example, as is the case for an array of wells in an otherwise continuous surface. The separation provided by an interstitial region can be partial or full separation. In embodiments, interstitial regions have a surface material that differs from the surface material of the wells. In some embodiments, interstitial regions have a surface material that is the same as the surface material of the wells.

In an embodiment, the spatially predefined locations are arranged in an array disposition.

An "array" is an ordered plurality of items. The term can refer to an ordered plurality of oligonucleotides, or the ordered array linked to a solid surface that is optionally planar, "ordered" refers to known locations on the array and is not intended to indicate a particular alignment of the items, though in some embodiments the items can be in a grid. In an embodiment, the capture probes are arranged in a 2D array.

Neighboring spatially predefined locations within the solid support (e.g., array) can be discrete one from the other in that they do not overlap. Accordingly, the spatially predefined locations can be adjacent to each other or separated by a gap (e.g., an interstitial space). In some embodiments where spatially predefined locations are spaced apart, neighboring spatially predefined locations can be separated, for example, by a distance of less than 10 µm, 5 µm, 1 µm, 0.9 µm, 0.8 µm, 0.7 µm, 0.6 µm, 0.5 µm or less. The layout of spatially predefined locations on an array can also be understood in terms of center-to-center distances between neighboring spatially predefined locations. A solid support useful in the invention can have neighboring spatially predefined locations with center-to-center spacing of less than about 10 µm, 5 µm, 1 µm, 0.9 µm, 0.8 µm, 0.7 µm, 0.6 µm, 0.5 µm, 0.4 µm or less. In some embodiments, the solid support has neighboring spatially predefined locations with center-to-center spacing of less than about 5 µm. In some embodiments, the solid support has neighboring spatially predefined locations with center-to-center spacing of less than about 1 µm. In some embodiments, the solid support has neighboring spatially predefined locations with center-to-center spacing of less than about 0.9 µm. In embodiments, the solid support has neighboring spatially predefined locations with center-to-center spacing of less than about 0.8 µm. In embodiments, the solid support has neighboring spatially predefined locations with center-to-center spacing of less than about 0.7 µm. In embodiments, the solid support has neighboring spatially predefined locations with center-to-center spacing of less than about 0.6 µm. In embodiments, the solid support has neighboring spatially predefined locations with center-to-center spacing of less than about 0.5 µm. In embodiments, the solid support has neighboring spatially predefined locations with center- to-center spacing of less than about 0.4 µm. Furthermore, it will be understood that the distance values described above and elsewhere herein can represent an average distance between neighboring spatially predefined locations of a solid support. As such, not all neighboring spatially predefined locations need to fall in the specified range unless specifically indicated to the contrary, for example, by a specific statement that the distance constitutes a threshold distance between all neighboring spatially predefined locations of an array.

Methods of making, using, and analyzing supports in which different capture probes are immobilized at spatially predefined locations (e.g., microarrays) are well known in the art. See, e.g., Baldi et al. (2002) DNA Microarrays and Gene Expression: From Experiments to Data Analysis and Modeling, Cambridge University Press; Beaucage (2001) "Strategies in the preparation of DNA oligonucleotide arrays for diagnostic applications" Curr Med Chem 8:1213-1244; Schena, ed. (2000) Microarray Biochip Technology, pp.19-38, Eaton Publishing; technical note "Agilent SurePrint Technology: Content centered microarray design enabling speed and flexibility" available on the web at chem.agilent.com/temp/rad01539/00039489.pdf; and references therein. Arrays of presynthesized polynucleotides can be formed (e.g., printed), for example, using commercially available instruments such as a GMS 417 Arrayer (Affymetrix, Santa Clara, CA). Alternatively, the polynucleotides can be synthesized at the selected positions on the solid support.

In some embodiments, the support has a plurality' of sites comprising at least 2, at least 3, at least 4, at least 5, at least 6, at least 7 or at least 8 sites. In those cases wherein one or more of the capture probes are used at different locations within the support, then the support comprises at least 10, at least 20, at least 30, at least 40 or more sites. In some embodiments, the spatially predefined location at which the capture probes are located has an area in the range of from 0.25 µm² to 1000 µm², or from 1 µm² to 1000 µm², or from 10 µm² to 1000 µm², or from 100 µm² to 1000 µm². In some embodiments, the amount of capture probes added at each of the spatially predefined locations is at least 10⁻⁶ fmol, or at least 10³ fmol, or at least 1 fmol, or at least 1 pmol, In some embodiments, the number of capture probes at each spatially predefined location is in the range of from 1000 molecules to 10⁶ molecules, or from 1000 molecules to 10⁹ molecules, or from 1000 molecules to 10¹² molecules. In some embodiments, the array is on a flow cell.

Once the amplicons are immobilized at the spatially predefined positions, they can be detected based on the presence of the detectable label at one or more of the spatially predefined positions, wherein if the label is detected at given position, then it is indicative that the sample contains the target nucleic acid which forms part of the tailed amplicon containing a sequence which is complementary to the sequence of the capture probe.

In an embodiment, the detectable label is detected indirectly using a nanoparticle that contains a ligand that binds specifically to the detectable tag present in the amplicons and then detect the nanoparticle that is associated to the immobilized amplicons. When a nanoparticle is used, it can be detected by the presence of a label which is adsorbed or chemically attached onto a surface of the nanoparticle, or by directly measuring a property of the material from which the nanoparticle is made.

The nanoparticle can contain any label detectable by spectroscopic, photochemical, biochemical, immunochemical, electrical, optical, or chemical means. Example detectable labels include, but are not limited to, fluorescent nanoparticles (e.g., quantum dots (Qdots)), metal nanoparticles (e.g., gold nanoparticles), metal-oxide nanoparticles, fluorescent dyes (e.g., fluorescein, texas red, rhodamine, green fluorescent protein, and the like, see, e.g., Molecular Probes, Eugene, Oreg., USA), radiolabels (e.g., ³H, ¹²⁵I, ³⁵S, ¹⁴C, ³²P, ⁹⁹TC, ²⁰³Pb, ⁶⁷Ga, ⁶⁸Ga, ⁷²As, ¹¹¹In ^{113m}In, ⁹⁷Ru, ⁶²Cu, ⁵²Fe, ^{52m}Mn, ⁵¹cr, ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²pr, ¹⁴³pr, ¹⁹⁸Au, ¹⁹⁹Au, ¹⁶¹Tb, ¹⁰⁹pd, ¹⁶⁵Dy, ¹⁴⁹pm, ¹⁵¹pm ¹⁵³Sm, ¹⁵⁷Gd, ¹⁵⁹Gd, ¹⁶⁶Ho, ¹⁷²TM, ¹⁶⁹Y b, ¹⁷⁵Yb, ¹⁷⁷Lu, ¹⁰⁵Rh, ¹¹¹Ag , and the like), enzymes (e.g., horse radish peroxidase, alkaline phosphatase and others commonly used in an ELISA), various colorimetric labels, magnetic or paramagnetic labels (e.g., magnetic and/or paramagnetic nanoparticles), spin labels, radio-opaque labels, and the like.

In some embodiments, the nanoparticle can be colorimetric, fluorescent, radioactive, magnetic, or enzymatic. In some embodiments, the nanoparticle is a gold nanoparticle or gold nanoshell that is detected through color. For example, the gold nanoparticle or gold nanoshell can have an absorbance wavelength of lower than or equal to 500 nm, lower than or equal to 525 nm, lower than or equal to 550 nm, lower than or equal to 575 nm, lower than or equal to 600 nm. Detectable color can be that of the visible spectrum. In some embodiments, the color is red, green, orange, purple, blue, or a combination thereof.

The nanoparticle can include a number of different materials suitable for the disclosed devices. For example, the detection nanoparticle can include a gold nanoparticle, a gold nanoshell, a colloidal carbon, or a latex bead. In some embodiments, the nanoparticle includes a gold nanoparticle or a gold nanoshell. In some embodiments, the detection nanoparticle is a gold nanoshell. In some embodiments, the detection nanoparticle is a gold nanoparticle. In some embodiments, the detection nanoparticle is a gold nanoshell or gold nanoparticle having an absorbance wavelength of lower than or equal to 600 nm. For example, the gold nanoshell or gold nanoparticle can have an absorbance wavelength of about 500 nm to about 600 nm.

The nanoparticle to be used in the detection step can have varying diameters. For example, the detection nanoparticle can have a diameter of about 10 nm to about 500 nm, such as about 50 nm to about 400 nm, about 100 nm to about 300 nm, about 125 nm to about 200 nm, about 10 nm to about 300 nm, about 10 nm to about 200 nm, or about 10 nm to about 100 nm. In some embodiments, the detection nanoparticle has a diameter of greater than or equal to 10 nm, greater than or equal to 20 nm, greater than or equal to 30 nm, greater than or equal to 40 nm, greater than or equal to 50 nm, greater than or equal to 75 nm, of greater than or equal to 100 nm, greater than or equal to 110 nm, greater than or equal to 120 nm, greater than or equal to 130 nm, greater than or equal to 140 nm, greater than or equal to 150 nm, greater than or equal to 175 nm, or greater than or equal to 200 nm. In some embodiments, the detection nanoparticle has a diameter of less than or equal to 500 nm, less than or equal to 400 nm, less than or equal to 350 nm, less than or equal to 300 nm, less than or equal to 250 nm, less than or equal to 200 nm, less than or equal to 175 nm, or less than or equal to 150 nm. In some embodiments, the detection nanoparticle is about 150 nm.

As it is used herein, the term "support" is an inert solid support which has a high surface area to enable a high adsorption capacity of nucleic acid and refers to a wide variety of materials including solids, semi-solids, gels, films, membranes, meshes, felts, composites, particles, and the like typically used to sequester molecules, and more specifically refers to an insoluble material with which nucleic acid can be associated. In a particular embodiment, the support is the test region of a lateral flow device.

In another embodiment, the detection of the amplicons generated in step (i) of the multiplexed detection method, once they are bound to the support in step (ii) are detected using nanoparticles which are coupled with a ligand that specifically binds the detectable tag present in the amplicon, wherein the particle is selected from the group comprising: metal nanoparticles, metal-oxide nanoparticles, latex particle, magnetic beads, carbon nanoparticle or quantum dots.

The term "specifically binds", as used herein, refer to the recognition and binding of two entities (for example, the detectable tag present in the amplicons and the ligand attached to the particles) in the absence of substantially recognition and binding other molecules in a sample. In some embodiments, a ligand can have, for example, an affinity for the detectable tag which is at least 10, 100, 1000 or 10,000 times greater than the affinity of the ligand for another reference molecule in a sample. In some embodiments the ligand can have a detectable tag binding dissociation constant (K_{D}) between about 0.01 nM and about 100 nM, or any value in between such as 0.2 nM.

Exemplary ligands include, but are not limited to, one member of a binding pair, nucleic acids, nucleosides and nucleotides, vitamins, hormones, proteins, peptides, peptidomimetics, amino acids, monosaccharides, disaccharides, oligosaccharides, polysaccharides, lipopolysaccharides, polyols, receptors, ligand for a receptor, and the like. In some embodiments of any one of the aspects described herein, the capture ligand is one member of a binding pair. As used herein, the term "binding pair" refers to a pair of moieties that specifically bind each other with high affinity, generally in the low micromolar to picomolar range. Non-limiting examples of binding pairs include antigen-antibody (including antigen- binding fragments or derivatives thereof), biotin-avidin, biotin-streptavidin, biotin-neutravidin (or other variants of avidin that bind biotin), two complementary nucleic acid strands, receptor-ligand, and the like. Additional molecule for binding pair can include, neutravidin, strep-tag, strep-tactin and derivatives, and other peptide, hapten, dye-based tags-anti-Tag combinations such as SpyCatcher-SpyTag, His-Tag, Fc Tag, Digitonin, GFP, FAM, haptens, SNAP-TAG. HRP, FLAG, HA, myc, glutathione S-transferase (GST), maltose binding protein (MBP), small molecules, and the like.

Therefore, in another particular embodiment, the authors of the present invention have observed that the method defined in the third aspect of the invention can be carried out in a lateral flow device, the device comprising:
(i) a sample loading region which contains a porous substrate in which the metal nanoparticles, metal-oxide nanoparticles, latex particle, magnetic beads, carbon nanoparticle or quantum dots coupled with a ligand that specifically binds the detectable tag are adsorbed,
(ii) a test region in which the capture probes are immobilized at the spatially predefined positions,
(iii) a channel which define a flow path between the sample loading region and the test region
wherein the sample is applied to the sample loading region under conditions adequate for the flow through the channels of the amplicons within the sample and of the metal nanoparticles, latex particle, magnetic beads, carbon nanoparticle or quantum dots coupled with a ligand that specifically binds the detectable tag into the test region and wherein the detection is carried out in the spatially predefined regions within the test region.

The device comprises a sample loading region which contains a porous substrate. The term "porous substrate" refers to a substrate possessing or full of pores, wherein the term "pore" refers to a minute opening or microchannel by which matter may be either absorbed or passed through. Particularly, where the pores allow passing-through of matter, the substrate is likely to be permeable.

The lateral flow device includes a porous substrate. The porous substrate includes a sample zone and a detection zone. The porous substrate can further include other zones as needed and depending on the application. The porous substrate can allow a mobile phase (e.g., a liquid sample) to flow through it by capillary action from the sample zone to the detection zone where a detectable signal, such as color changes or color differences on the device, may be generated to indicate the presence or absence of the target analyte. Accordingly, the porous substrate can define a flow path through which a sample added to the sample zone flows under capillary action downstream from the sample zone into the detection zone. As used herein, the term "capillary action" or "capillarity" refers to the process by which a molecule is drawn across the porous substrate due to such properties as surface tension and attraction between molecules.

The porous substrate can be made from any material sufficiently porous that can allow for capillary flow and detection of an analyte suitable for the disclosed devices. The porous substrate can include sintered glass or sintered ceramic, mineral, cellulose, fiberglass, nitrocellulose, polyvinylidene fluoride, nylon, charge modified nylon, polyethersulfone, or combination thereof. In some embodiments, the porous substrate includes sintered glass or sintered ceramic, mineral, cellulose, fiberglass, nitrocellulose, polyvinylidene fluoride, nylon, charge modified nylon, or polyethersulfone. In some embodiments, the porous substrate includes cellulose, fiberglass, or nitrocellulose. In some embodiments, the porous substrate includes nitrocellulose.

The term "region", as it is used herein, refers to both a specific portion of a sequence or a specific part of the lateral flow device.

The porous substrate includes a sample zone. The sample zone is configured to receive a sample. The sample once added to the sample zone can travel through the porous substrate toward the detection zone through capillary action. Accordingly, the detection zone is downstream from the sample zone.

In some embodiments, the sample zone includes a sample pad disposed on the porous substrate, in the porous substrate, or both. The sample pad may act as a filter that can remove debris, contaminants, and other unwanted substances present in the sample. The sample pad can include stored dried reagents (e.g., non-covalently bound) that can aid the detection of the analyte. For example, the dried reagents can adjust pH, ionic strength, and other properties of the sample fluid. Example materials that can be used for the sample pad include, but are not limited to, cellulose, nitrocellulose, fiberglass, cotton, woven or nonwoven paper, etc. Example reagents on the sample pad may include, but are not limited to, surfactants such as Triton X-100, Tween 20, or sodium dodecyl sulfate, etc.; polymers such as polyethylene glycol, poloxamer, polyvinylpyrrolidone (PVP), etc.; buffers such as phosphate-buffered saline, 4-(2-hydroxyethyl)-1-piperazineethanesulfonic acid (HEPES), Tris(hydroxymethyl) aminomethane (Tris), sodium borate, TRICINE, etc.; proteins such as albumin, etc.; enzymes such as protease, etc.; salts such as sodium chloride, sodium phosphate, sodium cholate, potassium phosphate, etc. The reagents can be applied to the sample pad by, e.g., soaking the material in the reagent solution, or through wicking the membrane via capillary flow. The treated sample pad can be dried by, e.g., air dry, heating at elevated temperatures, vacuum, or lyophilization.

Example materials that can be used for the conjugate pads include, but are not limited to, cellulose, nitrocellulose, fiberglass, cotton, woven or nonwoven paper, etc. In some embodiments, the conjugate pad for the detection nanoparticle is downstream from the conjugate pad for the control nanoparticle. In some embodiments, the conjugate pad(s) are downstream from the sample pad. The nanoparticles can be present in the sample pad and/or conjugate pad(s) as dried reagents (e.g., non-covalently bound). The nanoparticles can be applied to the sample pad and/or conjugate pad by, e.g., dipping the material in the reagent solution, or through spray coating the membrane. The treated sample pad and/or conjugate pad can be dried by, e.g., air dry, heated at adequate temperatures, vacuum, or lyophilization. Upon sample fluid reaching the sample pad and/or conjugate pad(s), the nanoparticles can be rehydrated. After rehydrating, the nanoparticles can interact with molecules of the sample and can travel along the porous substrate along with the fluid sample.

The porous substrate includes a detection zone. The detection zone is configured to detect a signal, e.g., from the different amplicon at the spatially predefined locations, from which the presence and/or amount of the amplicons to be detected. The sample, once added to the sample zone, can travel through the porous substrate toward the detection zone through capillary action. From the sample zone to the detection zone, the sample fluid can resuspend the detection nanoparticle, which can allow the detection nanoparticle to interact with the analyte if present. After the sample fluid has entered the detection zone, the amplicons can interact with capture probes present in the spatially predefined locations, which can allow for detection of signal denoting the presence and/or amount of the amplicon if a signal is detected in said predefined location within the support.

The detection zone includes at least two spatially predefined locations, each of them containing a specific capture probe. The detection zone can include 2 spatially predefined locations, 3 spatially predefined locations, 4 spatially predefined locations, 5 spatially predefined locations, 6 spatially predefined locations, 7 spatially predefined locations or 8 spatially predefined locations.

The spatially predefined locations can vary in distance in between each other. In some embodiments, a spatially predefined location is located at least 1 mm, at least 2 mm, at least 3 mm, at least 4 mm, at least 5 mm, at least 6 mm, at least 7 mm, or more from another spatially predefined location. In some embodiments, more than one spatially predefined location contains the same capture probe, which allows to detect the same amplicon at different predefined locations.

In some embodiments, the device contains a control region. This region is capable of binding those nanoparticles that are not bound to the immobilized amplicons by means of interaction with a second capture reagent present in the control region. In some embodiments, wherein the nanoparticle binds to the detectable tag by means of an antibody which is capable of specifically binding to the detectable tag, then the second capture reagent present in the control zone is a second antibody which is capable of specifically binding to the antibody forming part of the nanoparticle.

Immobilisation of the capture probes to the surface of the detection zone at the spatially predefined locations may be performed using surface chemistry. Nucleic acids can be readily immobilised by forming bonds via amine, sulfhydryl or cyanide groups (tethered to one end of nucleic acid). Blocking reagents such as Tween 20, BSA or casein may be used to block non-specific binding of the analyte, which may be performed after immobilisation.

The term "conditions adequate for the flow", as it is used herein, means those conditions known by the skilled person by which a nucleic acid sequences can flow. Illustrative non-limitative examples of conditions adequate for the hybridization are shown in the Examples.

Suitable instruments, software, and the like for analyzing arrays to distinguish selected positions on the solid support and to detect the presence or absence of a label (e.g., a fluorescently labeled label probe) at each position are commercially available. For example, microarray readers are available, e.g., from Agilent Technologies (Palo Alto, CA), Affymetrix (Santa Clara, CA), and Zeptosens.

In a certain embodiment, all the terms and embodiments described in any of the previous aspects of the invention are equally applicable to the third aspect of the invention.

### Set of probes

In a fourth aspect, the invention relates to a set of probes comprising different types of probes, wherein each type of probe contains a region having a substantial sequence complementary to barcode region wherein the barcode regions have a sequence as defined in any of SEQ ID NO:1 to 8 or a variant thereof capable of specifically hybridizing to a nucleic acid having a sequence which are complementary to SEQ ID NO:1 to 8 and wherein the region having a sequence complementary to a type of barcode region in each type of capture probe is different from that in the other capture probes of the set. In a particular embodiment, the capture probe contains 16 nucleotides of which 14 of them are complementary to the barcode region.

As referred to herein, "substantially complementary" refers to nucleotide sequences that can hybridize with each other under suitable hybridization conditions. Hybridization conditions can be altered to tolerate varying amounts of sequence mismatch within complementary nucleic acids that are substantially complementary. Substantially complementary portions of nucleic acids that can hybridize to each other can be 75 percent or more, 76 percent or more, 77 percent or more, 78 percent or more, 79 percent or more, 80 percent or more, 81 percent or more, 82 percent or more, 83 percent or more, 84 percent or more, 85 percent or more, 86 percent or more, 87 percent or more, 88 percent or more, 89 percent or more, 90 percent or more, 91 percent or more, 92 percent or more, 93 percent or more, 94 percent or more, 95 percent or more, 96 percent or more, 97 percent or more, 98 percent or more or 99 percent or more complementary to each other. In some embodiments substantially complementary portions of nucleic acids that can hybridize to each other are 100 percent complementary. Nucleic acids, or portions thereof, that are configured to hybridize to each other often include nucleic acid sequences that are substantially complementary to each other. As described herein, the complementarity of sequences may be partial, in which only some of the nucleic acids match according to base pairing, or complete, where all the nucleic acids match according to base pairing. Thus, two sequences that are complementary to each other, may have a specified percentage of nucleotides that complement one another (e.g., about 60 percent, preferably 65 percent, 70 percent, 75 percent, 80 percent, 85 percent, 90 percent, 91 percent, 92 percent, 93 percent, 94 percent, 95 percent, 96 percent, 97 percent, 98 percent, 99 percent, or higher complementarity over a specified region). In embodiments, two sequences are complementary when they are completely complementary (e.g., having 100 percent complementarity). In embodiments, sequences in a pair of complementary sequences form portions of a single polynucleotide with non-base-pairing nucleotides (e.g., as in a hairpin or loop structure, with or without an overhang) or portions of separate polynucleotides. In embodiments, one or both sequences in a pair of complementary sequences form portions of longer polynucleotides, which may or may not include additional regions of complementarity.

In another particular embodiment, the capture probe further contains an additional spacer region.

In a particular embodiment, the additional spacer region has the same sequence in all the different types of capture probes. In a preferred embodiment, the spacer region is located 3' with respect to the region complementary to the barcode region. In a preferred embodiment the spacer region is an oligoT, oligoC, oligoG, oligoU or oligoA sequence. In a more preferred embodiment, the additional spacer region is an oligoT or oligoA sequence. In a particular embodiment, the oligoT or oligoA sequence contains about 15 T or A nucleotides.

In another particular embodiment, the probes are immobilized on a support and wherein each type of probe is immobilized at a spatially predefined region in the support. More preferably, if the probes further contain an additional spacer region, then the probes are immobilized through the end of the capture probe which is located closer to the spacer region.

All the terms and embodiments described in any of the previous aspects of the invention are equally applicable to the fourth aspect of the invention.

### Method for the capture of different types of nucleic acid

In a fifth aspect, the invention relates to a method, hereinafter "third method of the invention", for the capture of a plurality of different types of nucleic acid present in a sample. In the third method of the invention, Method for the capture of a plurality of different types of nucleic acid present in a sample wherein each type of nucleic acid within the sample contains a barcode sequence as defined in any of SEQ ID NO:1 to 8 or a variant thereof capable of specifically hybridizing to a nucleic acid having the sequence of SEQ ID NO:1 to 8 and wherein the barcode region in each type of nucleic acid is different from the barcode regions in the other types of nucleic acids in the sample, the method comprising contacting the sample with a set of probes as defined in any of claims 27 or 28, said contacting being carried out under conditions adequate for the hybridization between the barcode regions present in each type of nucleic acid and the sequences complementary to said barcode region in each type of capture probes.

The term "plurality" applied to the number of different types of nucleic acid present in a sample preferably comprises at least 1, 2, 3, 4, 5, 6, 7, 8, 9, 10 or more.

In a certain embodiment, all the terms and embodiments described in any of the previous aspects of the invention are equally applicable to the fifth aspect of the invention.

### Set of primers

In a sixth aspect, the invention relates to a set of primers wherein the set of primers contains at least two types of primers being each type of primer specific for a different target nucleic acid wherein each given type of primer contains a target-specific region which is capable of specifically hybridizing to the target nucleic acid within the series of different target nucleic acids and a barcode region which comprises a sequence selected from the group consisting of SEQ ID NO: 1 to 8, wherein the barcode region within the primer in a type of primer pairs is different from the barcode regions within the primers in the other types of primers.

The terms "target-specific region", "barcode region" and "spacer" have been previously defined in relation to the first aspect of the invention and are incorporated herein by reference.

In some embodiments, the barcode region, refers to a forward primer region which comprises a sequence selected from the group consisting of SEQ ID NO: 1 to 8 as defined below

**Table 1: Sequences of the barcode regions**

| **Name** | **Sequences** |
|---|---|
| SEQ ID NO: 1 | GTT TTC CCA GTC AC |
| SEQ ID NO: 2 | TTG GTC GTG GTG GT |
| SEQ ID NO: 3 | CAT CTT TAT TAG CT |
| SEQ ID NO: 4 | GTT TAG CAC ATC AG |
| SEQ ID NO: 5 | AAC GCT CAC CCA TG |
| SEQ ID NO: 6 | CGG AGT TGA GGT GA |
| SEQ ID NO: 7 | TCT AGT AGT GGA TC |
| SEQ ID NO: 8 | CTA TGG TGA ACG AA |

In some embodiments:
- the barcode region contains a sequence which is a variant of SEQ ID NO:1 and which is capable of specifically hybridizing to a nucleic acid having a sequence which is complementary to SEQ ID NO:1.
- the barcode region contains a sequence which is a variant of SEQ ID NO:2 and which is capable of specifically hybridizing to a nucleic acid having a sequence which is complementary to SEQ ID NO:2.
- the barcode region contains a sequence which is a variant of SEQ ID NO:3 and which is capable of specifically hybridizing to a nucleic acid having a sequence which is complementary to SEQ ID NO:3.
- the barcode region contains a sequence which is a variant of SEQ ID NO:4 and which is capable of specifically hybridizing to a nucleic acid having a sequence which is complementary to SEQ ID NO:4.
- the barcode region contains a sequence which is a variant of SEQ ID NO:5 and which is capable of specifically hybridizing to a nucleic acid having a sequence which is complementary to SEQ ID NO:5.
- the barcode region contains a sequence which is a variant of SEQ ID NO:6 and which is capable of specifically hybridizing to a nucleic acid having a sequence which is complementary to SEQ ID NO:6.
- the barcode region contains a sequence which is a variant of SEQ ID NO:7 and which is capable of specifically hybridizing to a nucleic acid having a sequence which is complementary to SEQ ID NO:7 and/or
- the barcode region contains a sequence which is a variant of SEQ ID NO:8 and which is capable of specifically hybridizing to a nucleic acid having a sequence which is complementary to SEQ ID NO:8.

In a particular embodiment, the target-specific region within the primer is located 3' with respect to the barcode region. In a more particular embodiment, the target-specific region and the barcode region within the primer from each type of primer pair are connected by a spacer group.

In a particular embodiment, the spacer group is a polyethylene group. In another particular embodiment, the spacer group has the following structure 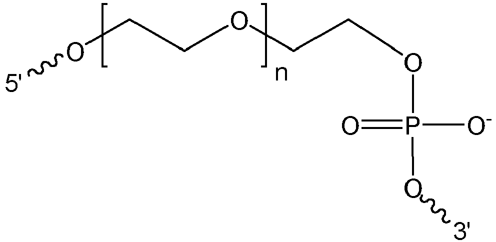 wherein n is from 0 to 18. More preferably, the spacer group has the structure

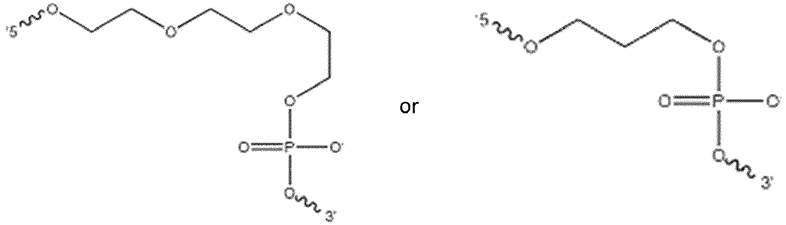

In a particular embodiment, the length of the barcode region within each type of primer pair is of between 12 and 18 nucleotides. More preferably, the length of the barcode region is of 14 nucleotides.

In another particular embodiment, all the terms and embodiments described in any of the previous aspects of the invention are equally applicable to the sixth aspect of the invention.

### Kits and uses thereof

In a seventh aspect, the invention relates to a kit comprising a set of primer pairs as defined in the first aspect and a set of capture probes as defined in the fourth aspect.

As it is used herein, the term "kit" refers to a product containing the different reagents necessary for carrying out the uses and methods of the invention which is packed so as to allow their transport and storage. Materials suitable for packing the components of the kit include crystal, plastic (e.g., polyethylene, polypropylene, polycarbonate), bottles, vials, paper or envelopes.

All the terms and embodiments described in any of the previous aspects of the invention are equally applicable to the seventh aspect of the invention.

Also disclosed herein are kits that can be used for, e.g., detecting an analyte using the disclosed lateral flow devices. The kit can include a disclosed device, and one or more packages, receptacles, labels, or instructions for use. The kit may include at least one buffer. The kit may also include other components to facilitate using the device and methods thereof. Examples of such components include, but are not limited to, one or more additional reagents, such as one or more dilution buffers; one or more reconstitution solutions; one or more wash buffers; one or more storage buffers, one or more control reagents, (one or more additional component(s), such as a sample collection device (e.g., a syringe, cotton swab, tongue depressor, and the like), and the like. Components (e.g., reagents, components, etc.) may also be provided in a form that is usable in a particular assay, or in a form that requires addition of one or more other components before use (e.g. in concentrate or lyophilized form). Suitable buffers include, but are not limited to, phosphate buffered saline, sodium carbonate buffer, sodium bicarbonate buffer, borate buffer, Tris buffer, MOPS buffer, HEPES buffer, and combinations thereof. The choice of buffers and reagents will depend on the particular application, e.g., setting of the assay (point-of-care, research, clinical), analyte(s) to be assayed, the detection moiety used, etc. Such components may be provided individually or in combination(s) and may be provided in any suitable container such as a vial, a bottle, box, or a tube.

The kit may also include a packaging configured to contain the device and other components. The packaging may be a sealed packaging, such as a sterile sealed packaging. By "sterile" it is meant that there are substantially no microbes (such as fungi, bacteria, viruses, spore forms, etc.). In some embodiments, the packaging may be configured to be sealed, e.g., a water vapor-resistant packaging, optionally under an airtight and/or vacuum seal.

Following construction of the device, it can be optionally dried, e.g., by mild desiccation, blow drying, lyophilization, or exposure to ambient air at ambient temperature, for a time sufficient for the article to be dry or at least macroscopically dry. Once the device is dry or at least macroscopically dry, it may be sealed in a container (e.g., such as an impermeable or semipermeable polymeric container) in which it can be stored and shipped to a user. Once sealed in a container, the device may have, in some embodiments, a shelf life of at least 2 to 4 months, or up to 6 months or more, when stored at a temperature of 25 degrees centigrade (e.g., without loss of more than 20 percent, 30 percent or 50 percent of binding activity).

In addition to above-mentioned components, a subject kit can further include instructions for using the components of the kit to practice the disclosed methods. The instructions for practicing the methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or sub-packaging) etc. In some embodiments, the instructions are present as an electronic storage data file present on a suitable computer readable storage medium, e.g., CD-ROM, diskette, flash drive, etc. In some other embodiments, the actual instructions are not present in the kit, but means for obtaining the instructions from a remote source, e.g., via the internet, are provided. An example of this embodiment is a kit that includes a web address where the instructions can be viewed and/or from which the instructions can be downloaded. As with the instructions, this means for obtaining the instructions is recorded on a suitable substrate.

In some embodiments, the kit includes instructions that correlate the number, intensity, or both of visible test lines with the concentration of the analyte in the sample. In some embodiments, the kit includes a reference card that correlates the number and intensity of visible test lines with the concentrations of the analyte in the sample. In some embodiments, the kit further includes a smart device (e.g., tablet, smart phone, etc.) lateral flow device reader for the device(s).

In another aspect, the invention relates to a use of a kit according to the seventh aspect in a method for the multiplexed detection of a series of target nucleic acids in a sample wherein the capture probes are immobilized in a support at spatially predefined locations. In a particular embodiment, the detection is carried out using a lateral flow chamber.

### Devices and uses thereof

In another embodiment, the invention relates to a device which contains the set of capture probes defined in the fourth aspect of the invention.

In an embodiment, the device is a lateral flow device.

The description of the devices, porous substrates, sample zones, detection zones, detection nanoparticles, control nanoparticles, test lines, control lines, and other components used in the method for multiplexed detection of nucleic acids are equally applicable to the devices as disclosed herein.

In another aspect, the invention relates to a use of a device according to the seventh aspect in a method for the multiplexed detection of a series of target nucleic acids in a sample wherein the capture probes are immobilized in a support at spatially predefined locations.

The invention is defined bellow by the following aspects:
1. A set of primer pairs for the multiplexed detection of a series of different target nucleic acids wherein the set contains at least two types of primer pairs being each type of primer pair specific for a different target nucleic acid wherein, for each given type of primer pair:
   (i) The forward primer contains a target-specific region which is capable of specifically hybridizing to a target nucleic acid within the series of different target nucleic acids and a barcode region which comprises a sequence selected from the group consisting of SEQ ID NO: 1 to 8 or a variant thereof capable of specifically hybridizing to a nucleic acid having a sequence which are complementary to SEQ ID NO.1 to 8 and,
   (ii) the reverse primer contains a target-specific region which is capable of specifically hybridizing to said target nucleic acid and which is labelled in its 5' end with a detectable tag
   wherein the barcode region within the forward primer in a type of primer pairs is different from the barcode regions within the forward primers in the other types of primer pairs.
2. The set according to aspect 1 wherein the target-specific region within the forward primer is located 3' with respect to the barcode region.
3. The primer set according to any of aspects 1 or 2 wherein the target-specific region and the barcode region within the forward primer from each type of primer pair are connected by a spacer and/or wherein the target-specific region and the detectable tag in the reverse primer from each type of primer pair are connected by a spacer group.
4. The primer set according to aspect 3 wherein the spacer group is a polyethyleneglycol group or alkyl group.
5. The primer set according to aspect 4 wherein the spacer group has the following structure 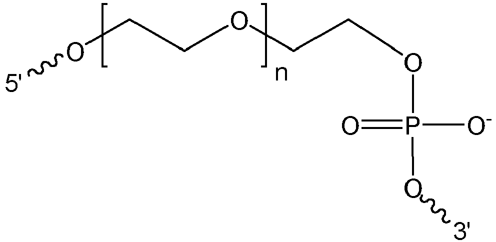 wherein n is from 0 to 18.
6. The primer set according to aspect 5 wherein the spacer group has the structure 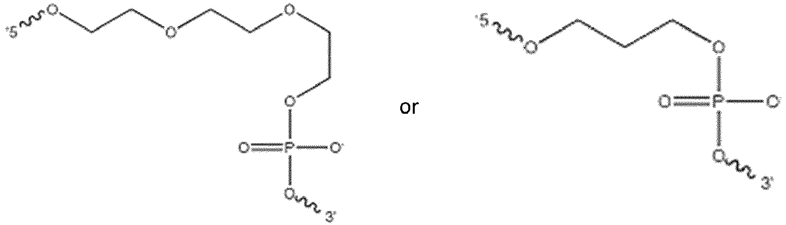
7. The primer set according to any of aspects 1 to 6 wherein the length of the barcode region in the forward primers within each type of primer pair is of between 12 and 20 nucleotides.
8. The primer set according to any of aspect 7 wherein the length of the barcode region in the forward primers within each type of primer pair is of 14 nucleotides.
9. The primer set according to any of aspects 1 to 8 wherein the detectable tag within the reverse primer in each primer pair is connected to the 5' end of the reverse primer.
10. The primer set according to any of aspects 1 to 9 wherein the detectable tag is selected from a group comprising:
   (i) 6-carboxyfluorescein (FAM)
   (ii) Biotin
   (iii) Digoxigenin
   (iv) Fluorescein
   (v) Oligonucleotide having a predefined sequence.
11. A method for the multiplexed amplification of a series of target nucleic acids in a sample comprising contacting the sample with a set of primer pair as defined in any of aspects 1 to 10 wherein the set of primers contains primer pairs containing forward and reverse primers specific for each of the target nucleic acids in the sample, wherein said contacting is carried out under conditions adequate for the amplification of the target nucleic acid thereby generating amplicons from each target nucleic acid, wherein each amplicon contains a tail comprising the sequence of the barcode region forming part of the specific forward primer and the detectable tag forming part of the specific reverse primer.
12. The method according to aspect 11 wherein the amplification is an isothermal amplification.
13. A method for the multiplexed detection of a series of target nucleic acids in a sample comprising the steps of:
   (i) Generating amplicons from each type of target nucleic acid in a sample by means of a method as defined in aspects 11 or 12,
   (ii) Contacting the amplicons formed in step (i) with a support, said support containing different types of capture probes immobilized each of them at spatially predefined positions within the support, wherein each type of capture probe contains a region which contains a sequence complementary to a type of barcode region present in the amplicons, said contacting being carried out under conditions adequate for the hybridization between the tail in each type of amplicon and the complementary barcode region in the capture probes
   wherein detection of the detectable label at one or more of the spatially predefined positions is indicative that the sample contains the target nucleic acid which forms part of the tailed amplicon containing a sequence which is complementary to the sequence of the capture probe.
14. The method according to aspect 13 wherein the detection of the immobilized amplicons is carried out using particle coupled with a ligand that specifically binds the detectable tag, wherein the particle is selected from the group comprising: metal nanoparticles, latex particle, magnetic beads, carbon nanoparticle or quantum dots.
15. The method according to aspect 13 wherein the capture probe contains 16 nucleotides of which 14 of them are complementary to the barcode region.
16. The method according to any of aspects 13 to 15 wherein the capture probes further contain a spacer region which connects the region complementary to the barcode sequence to the support.
17. The method according to aspect 16 wherein the spacer region is an oligoT or an oligoA sequence.
18. The method according to aspect 17 wherein the oligoT sequence contains about 15 thymidine nucleotides.
19. The method according to any of aspects 13 to 18 wherein the method is carried out in a lateral flow device, the device comprising
   (i) a sample loading region which contains a porous substrate in which the metal nanoparticles, latex particle, magnetic beads, carbon nanoparticle or quantum dots coupled with a ligand that specifically binds the detectable tag are adsorbed,
   (ii) a test region in which the capture probes are immobilized at the spatially predefined positions,
   (iii) a channel which defines a flow path between the sample loading region and the test region
   wherein the sample is applied to the sample loading region under conditions adequate for the flow through the channels of the amplicons within the sample and of the metal nanoparticles, latex particle, magnetic beads, carbon nanoparticle or quantum dots coupled with a ligand that specifically binds the detectable tag into the test region and wherein the detection is carried out in the spatially predefined regions within the test region.
20. A set of probes comprising different types of probes, wherein each type of probe contains a region having a sequence substantially complementary to barcode region wherein the barcode regions are as defined in any of SEQ ID NO:1 to 8 or a variant thereof capable of specifically hybridizing to a nucleic acid having a sequence which are complementary to SEQ ID NO.1 to 8 wherein the region having a sequence complementary to a type of barcode region in each type of capture probe is different from that in the other capture probes of the set.
21. The set according to aspect 20 wherein the capture probe contains 16 nucleotides of which 14 of them are complementary to the barcode region.
22. The set according to aspects 20 or 21 wherein the capture probe further contains an additional spacer region.
23. The set according to aspect 22 wherein the additional spacer region has the same sequence in all the different types of capture probes.
24. The set according to aspects 22 or 23 wherein the spacer region is located 3' with respect to the region complementary to the barcode region.
25. The set according to any of aspects 20 to 24 wherein the additional spacer region is an oligoT or oligoA sequence.
26. The set according to aspect 25 wherein the oligoT or oligoA sequence contains about 15 T or A nucleotides.
27. The set of probes according to any of aspects 20 to 26 wherein the probes are immobilized on a support and wherein each type of probe is immobilized at a spatially predefined region in the support.
28. The set of probes according to aspect 27 wherein, if the probes further contain an additional spacer region, then the probes are immobilized through the end of the capture probe which is located closer to the spacer region.
29. Method for the capture of a plurality of different types of nucleic acid present in a sample wherein each type of nucleic acid within the sample contains a barcode sequence as defined in any of SEQ ID NO:1 to 8 or a variant thereof capable of specifically hybridizing to a nucleic acid having the sequence of SEQ ID NO:1 to 8 and wherein the barcode region in each type of nucleic acid is different from the barcode regions in the other types of nucleic acids in the sample, the method comprising contacting the sample with a set of probes as defined in any of aspects 27 or 28, said contacting being carried out under conditions adequate for the hybridization between the barcode regions present in each type of nucleic acid and the sequences complementary to said barcode region in each type of capture probes.
30. A set of primers wherein the set of primers contains at least two types of primers being each type of primer specific for a different target nucleic acid wherein each given type of primer contains a target-specific region which is capable of specifically hybridizing to the target nucleic acid within the series of different target nucleic acids and a barcode region which comprises a sequence selected from the group consisting of SEQ ID NO: 1 to 8 or a variant thereof which is capable of specifically hybridizing to a nucleic acid having the sequence of SEQ ID NO;1 to 8, wherein the barcode region within the primer in a type of primer pairs is different from the barcode regions within the primers in the other types of primers.
31. The primer set according to aspect 30 wherein the target-specific region within the primer is located 3' with respect to the barcode region.
32. The primer set according to any of aspects 30 or 31 wherein the target-specific region and the barcode region within the primer from each type of primer pair are connected by a spacer group.
33. The primer set according to aspect 32 wherein the spacer group is a polyethylene group.
34. The primer set according to aspect 33 wherein the spacer group has the following structure 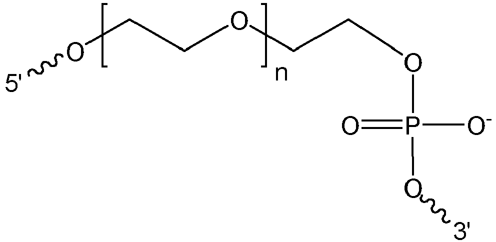 wherein n is from 0 to 18.
35. The primer set according to aspect 34 wherein the spacer group has the structure 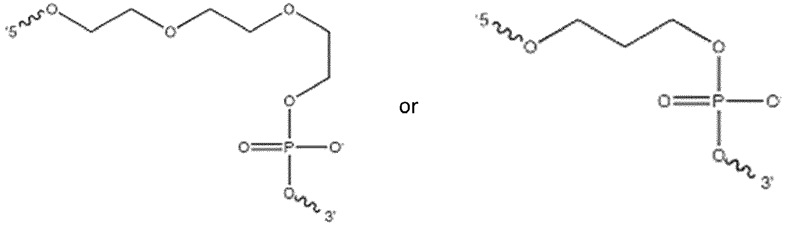
36. The primer set according to any of aspects 30 to 35 wherein the length of the barcode region within each type of primer pair is of between 12 and 18 nucleotides.
37. The primer set according to any aspect 36 wherein the length of the barcode region is of 14 nucleotides.
38. A kit comprising a set of primer pairs as defined in any of aspects 1 to 10 and a set of capture probes as defined in any of aspects 20 to 28.
39. A device comprising a set of capture probes as defined in any of aspects 20 to 28.
40. The device according to aspect 39 wherein the device is a lateral flow device.
41. Use of a kit according to aspect 38 or of the device according to aspects 39 or 40 in a method for the multiplexed detection of a series of target nucleic acids in a sample wherein the capture probes are immobilized in a support at spatially predefined locations.
42. Use according to aspect 41 wherein the detection is carried out using a lateral flow chamber.

The invention is described below by means of the following examples which are to be construed as merely illustrative and not limitative of the scope of the invention.

### EXAMPLES

### Example 1: Design of the multiplex system

### 1. Overview

In this example is described the development of a functional multiplexed lateral flow test (mLFT).

The primer design took into consideration a couple of strategies to be able to detect the amplicon with a LF and increase the number of targets that can be detected in a LF strip. The strategy selected for detection of the amplicon consisted in one primer labeled with FAM and the other with a tail (Fig. 1). Consequently, the selected primers for each target were modified accordingly: the forward primer with a tail or barcode (random short DNA sequence generated) and the reverse primer with FAM. Therefore, DNA probes complementary to the tails were designed to work as TL and to be immobilized in the nitrocellulose.

Different designs of tails and TL were tested while targets were combined in groups for multiplex amplification. Optimization of the amplification was done in parallel with the optimization of the multiplexed LF test (mLFT).

A proof-of-concept multiplexed LFT (mLFT) was developed either with multiple printed lines (4 test lines and 1 control line) or a dot array (Fig. 2).

### 2. Materials and Methods

### 2.1. Materials

For the isothermal amplification: Gel loading Dye and Ladder Quick Load Purple from New England BioLabs (M0307, B7025S and N0557S), TBE UltraPure^{™} and Agarosa UltraPure^{™} from Invitrogen (15581-044 and 16500-100), GelGreen^{®} Nucleic Acid Gel Stain from minipcrbio^{®} (RG-1550-01), PCR grade water from VWR (E476), 10 mM TE from IDT.

For the LF platform: Cellulose fiber, laminated cards and glass fiber from Millipore (CFSP001700, HF000MC100 and GFDX000800, respectively); nitrocellulose membrane from MDI membrane technologies (CNPC-SS12-10 µm) ; tetrachloroauric (III) acid (HAuCl₄), sodium citrate, boric acid, sodium tetraborate, bovine serum albumin, sucrose and Tween 20; iridium (III) chloride, tris-buffered saline tablets, saline-sodium citrate buffer, betaine, formamide.

Primers and templates for the amplification, and Ab and probes for the LF platform are described in the following sections.

### 2.1.1. Equipment

Continuous Reagent Dispenser and Programmable Strip Cutter from Shanghai Kinbio Tech (HM8008 and ZQ2002, respectively); drying oven from Mestra (80118); UV-Vis Spectrophotometer from Molecular Devices (SpectraMax iD3); Thermoshaker from Labnet (EQN022); Biocen Centrifuge from Orto Alresta (model 22R); transmission electron microscope from JEOL (1210); SimpliAmp^{™} Thermal Cycler from Applied Biosystems (A24811); and blueGel^{™} electrophoresis with built-in transilluminator from minipcrbio^{®}(QP-1500-01).

### 2.2. Methods

### 2.2.1. Targets

Resistance determinants to be detected can be found in Table 2. To the list of these resistance determinants, one more target was added, the Internal Process Control (IPC), which is an armored control with a known stock concentration. The IPC was heated 75 °C for 5 min to release the genetic material before each amplification.

IPC consists of a DNA sequence packed inside a protein coat, which stabilizes and protects nucleic acids from nuclease degradation. IPC can be added to the sample being included in the workflow of the test to work as an internal control for extraction and detection in molecular assays.

**Table 2. List of targets to be detected plus the IPC.**

| **Target #** | **Name** |
|---|---|
| 1 | OXA-48 group |
| 2 | CTX-M-1 group |
| 3 | VIM group |
| 4 | KPC group |
| 5 | OXA-23 group |
| 6 | IMP group |
| 7 | NDM group |
| 8 | IPC (Internal Process Control) |

### 2.2.2. Isothermal amplification

A commercial kit was applied for RPA reaction. Manufacturer instructions were followed with some modifications. Briefly, each RPA reaction was set to a final volume of 25 µL and activated with 14 mM magnesium acetate. RPA was then performed on the thermal cycler at 37 °C for 25 min.

### 2.2.3. Agarose gel electrophoresis

A 3% agarose gel was prepared and dyed with GelGreen. To clean the amplification product before loading the sample, RPA products were heated at 65° for 10 min. Next, each RPA product was mixed with the Gel Loading Dye in the ratio indicated by the manufacturer and loaded in the corresponding well in the gel. In every electrophoresis gel a well was loaded with a ladder.

Then, the agarose gel electrophoresis was run for 45 min in the blueGel^{™} electrophoresis with built-in transilluminator at 48 V (not adjustable). After that time, instrument was stopped, and picture of the gel was taken using the transilluminator.

### 2.2.4. Design of the multiplexed LF platform

To increase the number of targets that can be detected in a single LF strip, ssDNA probes were generated (random short DNA sequence) as TL and labelled with an amino-group to be print in the detection pad.

This strategy implied labelling one of the primers with a short ssDNA sequence complementary to the corresponding TL (Table 3). The other primer could still be labelled with FAM and be captured by an anti-FITC Ab conjugated to nanoparticles (Table 4). Consequently, the selected primer pair for each target was modified accordingly: the forward primer with a tail and the reverse primer with FAM. This way, each of the amplicons would be detected as shown in Fig. 1.

As shown in Table 3, the following probes (anchored in the nitrocellulose support) were tested for a multiplexed system. For instance, in Fig. 3, it can be visualized the alignment of the capture probe with their corresponding primer tail.

**Table 3. Sequences of the first capture probes and the corresponding tails for the primer.**

| **Name** | **Sequence (5'-3')** | **Tail Sequence (5'-3')** |
|---|---|---|
| TL1 | GTCGTGACTGGGAAAACTTTTTTTTTTTTTTT | GTT TTC CCA GTC ACG AC |
| TL2 | ACCACCACGACCAATTTTTTTTTTTTTTT | TTG GTC GTG GTG GTG GTT T |
| TL3 | AGC TAA TAA AGA TGT TTT TTT TTT TTT TT | CAT CTT TAT TAG CT |
| TL4 | GCA CGT ATT GTG CAT TTT TTT TTT TTT TT | TGC ACA ATA CGT GC |
| TL5 | CTG ATG TGC TAA ACT TTT TTT TTT TTT TT | GTT TAG CAC ATC AG |

**Table 4. Antibodies used in the multiplexed LF platform.**

| **Cat ID** | **Supplier** | **Target** | **Isotype** | **Clonality** | **Position in the test** |
|---|---|---|---|---|---|
| 31242 | Invitrogen | FITC | Mouse IgG1 | Monoclonal | Nanoparticles |
| 31232 | Invitrogen | Antimouse | Goat IgG | Monoclonal | CL |

### 2.2.5. Multiplexed LF strips preparation

LF strips were assembled according to a protocol previously published (Parolo,C.; Sena-Torralba,A.; Bergua,J. F.; Calucho,E.; Fuentes-Chust,C.; Hu,L.; Rivas,L.; Álvarez-Diduk,R.; Nguyen,E. P.; Cinti,S.; Quesada-González, D.; Merkoçi,A. Tutorial: Design and Fabrication of Nanoparticle-Based Lateral-Flow Immunoassays .Nat.Protoc.2020,15(12),3788-3816), composed by the four typical pads.

Sample and absorbent pads are composed by cellulose membrane, being the first one treated with tris-buffered saline (TBS), 5 % BSA and 0.05% Tween 20.

Nanoparticles conjugated with antibodies are dry-stored in the glass fiber (i.e. the conjugate pad). In the detection pad, 25 µM of each Test amino-labeled DNA probes in TBS and 1 mg/mL of CL Ab were dispensed with the continuous reagent dispenser. Therefore 4 TL and 1 CL were printed. Finally, all the pads were assembled in the laminated card and LF strips were cut to 4 mm width.

### 2.2.6. Multiplexed LF assays

10 µL of the product amplification were added in 110 µL of TBS with 0.5 % Tween (running buffer) in a tube. Sample was placed in the sample pad and the results observed in the strip were read after 15 min.

### 3. Results

### 3.1. Capture probes

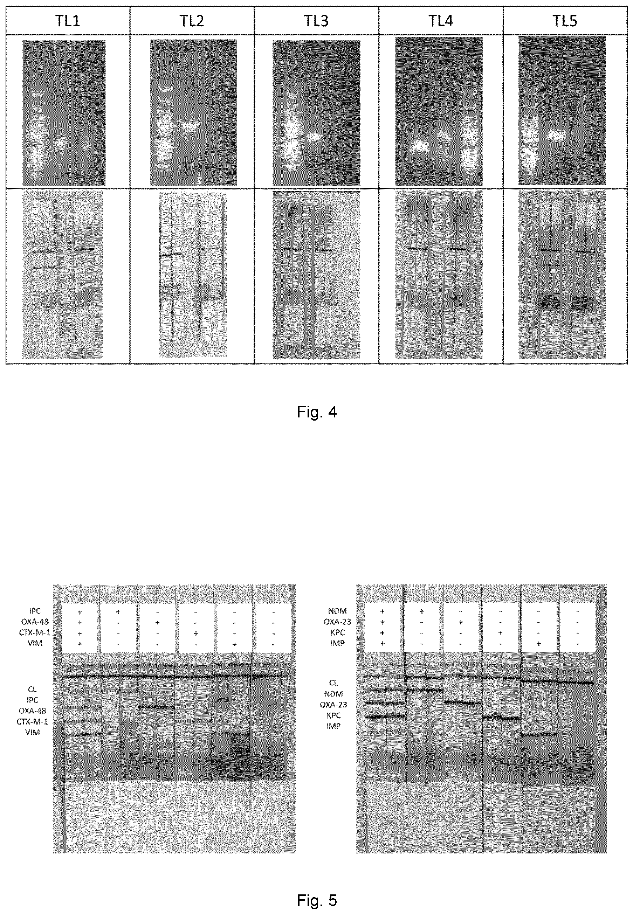

As depicted in Fig. 4, the correct amplification could be observed using the primers with the tails in an agarose gel and then the detection of the amplicon using lateral Flow strips with the capture probe printed on the TL for TL1, TL2, TL3 and TL5. With TL4 there was no recognition in any case despite the amplification product being present.

### 3.2. Multiplexed LF assays

The targets were successfully amplified in two main groups of four targets each in a quadruplex reaction (Table 5) and detected optically by the mLFT using multiple lines (Fig. 5). Sequences of TL and tails used can be found in Example 2.

**Table 5. Quadruplex composition and the corresponding TL for the amplicon detection.**

| **Quadruplex 1** | | **Quadruplex 2** | |
|---|---|---|---|
| **Target** | **TL** | **Target** | **TL** |
| IPC | 2 | NDM | 6 |
| OXA-48 | 1 | OXA-23 | 7 |
| CTX-M-1 | 3 | KPC | 8 |
| VIM | 5 | IMP | 9 |

As can be seen in Fig. 5, two different LF strips of multiple lines were developed, one for detection of the amplification products of quadruplex 1 and the second for the amplification products of quadruplex 2.

### Example 2: Array for detection of antibiotic resistance genes

### 1. Overview

The Example describes the use of oligos in a multiplexed array format for detection of antibiotic resistance genes.

In lateral flow (LF) devices, the detection capacity of multiple elements is limited by the size of the detection area (detection pad), and/or the number of markers that the different pads can store.

To avoid the need to find different labels and the consequent capture antibodies, different single-stranded DNA (ssDNA) probes were generated as bioreceptors to be placed on the detection pad (capture probes, TLs), and one of the primers for each amplification target was labeled with the complementary sequence (tail or barcode). The other primer was labeled with 6-carboxyfluorescein (FAM), resulting in an amplicon flanked on one side by an ssDNA tail and on the other side by a FAM (see Fig. 1). In this case FAM was used as a label, but other labels could be used as well.

In this way, the use of tailed primers allows the detection of multiple amplifications by hybridization through the capture probes in the detection zone and then the recognition by the antibody labeled with nanoparticles.

Therefore, the use of tailed primers avoids the need to find different labels and subsequent capture antibodies for detection of multiple elements by LF and avoids too the need for any post-amplification processing for the generation of ssDNA. Furthermore, the LF platform can be used for the detection of other targets by simply adding the corresponding tails to the designed specific primers. Next, the arrangement of these capture probes in the form of a microarray instead of in the typical test lines allows increasing the number of targets that can be detected per strip.

### 2. Materials and Methods

### 2.1. Materials

Primers and tails were purchased from Integrated DNA Technologies (IDT; Leuven, Belgium) and other materials used to perform the experiments are fully described in Example 1 (Materials and Methods section), except for the nitrocellulose membrane VIV902550R from Pall Life Science that was used instead for the array.

### 2.2. Methods

### 2.2.1. Capture probes

As shown in Table 3, the following probes (anchored in the nitrocellulose support) were used for the array. Each probe was assigned a target according to Table 3, and the corresponding target carried the complementary tail in one of the primers (Table 4).

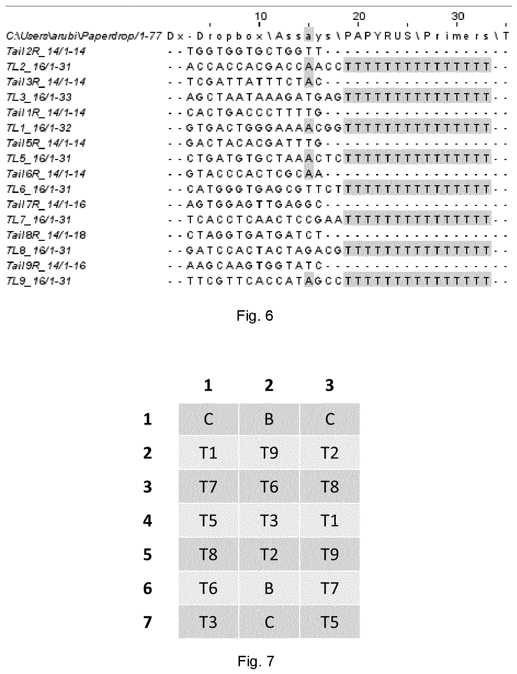

For instance, in Fig. 6, it can be visualized the alignment of the probe with their corresponding primer tail.

**Table 6. Sequences of the capture probes applied in the array and the target assigned.**

| **Name** | **Sequence (5'-3')** | **Target** |
|---|---|---|
| TL1_16 | GTG ACT GGG AAA ACG GTT TTT TTT TTT TTT T | OXA-48 |
| TL2_16 | ACC ACC ACG ACC AAC CTT TTT TTT TTT TTT T | IPC |
| TL3_16 | AGC TAA TAA AGA TGA GTT TTT TTT TTT TTT T | CTX-M-1 |
| TL5_16 | CTG ATG TGC TAA ACT CTT TTT TTT TTT TTT T | VIM |
| TL6_16 | CAT GGG TGA GCG TTC TTT TTT TTT TTT TTT T | NDM |
| TL7_16 | TCA CCT CAA CTC CGA ATT TTT TTT TTT TTT T | OXA-23 |
| TL8_16 | GAT CCA CTA CTA GAC GTT TTT TTT TTT TTT T | KPC |
| TL9_16 | TTC GTT CAC CAT AGC CTT TTT TTT TTT TTT T | IMP |

**Table 7. Sequences of the tails of the primers and the target assigned.**

| **Name** | **Sequence (5'-3')** | **Target** |
|---|---|---|
| Tail1 | GTT TTC CCA GTC AC | OXA-48 |
| Tail2 | TTG GTC GTG GTG GT | IPC |
| Tail3 | CAT CTT TAT TAG CT | CTX-M-1 |
| Tail5 | GTT TAG CAC ATC AG | VIM |
| Tail6 | AAC GCT CAC CCA TG | NDM |
| Tail7 | CGG AGT TGA GGT GA | OXA-23 |
| Tail8 | TCT AGT AGT GGA TC | KPC |
| Tail9 | CTA TGG TGA ACG AA | IMP |

### 2.2.2. Microarray preparation

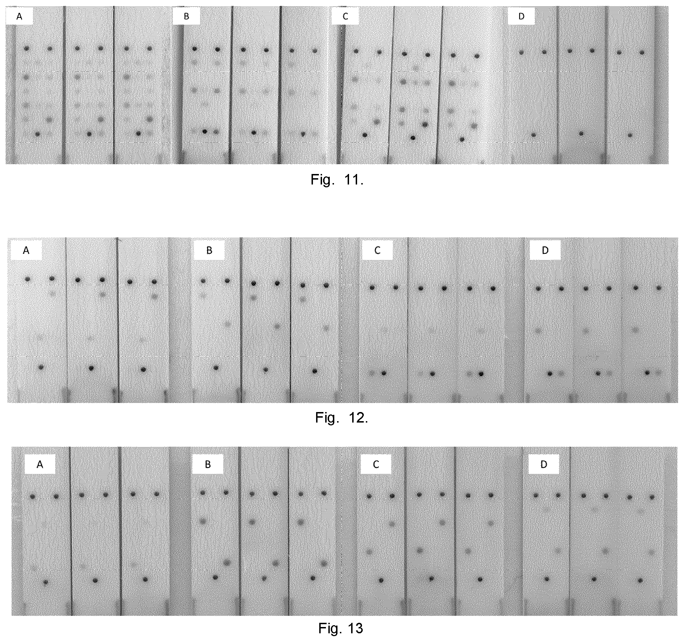

Printing conditions were as follows:
- Capture probe concentration: 50 µM
- Printing buffer: SSC 4x
- Example of a design printed can be seen in Fig. 13.
and the other pads were assembled as indicated in Example 1 (Materials and Methods section).

### 3. Results

### 3.1. Primer design

As mentioned, to amplify the targets one of the primers is labeled with FAM and the other one with a single-stranded DNA tail. To join the tail with the corresponding primer, 2 different spacers were tested, Spacer 9 and Spacer C3, so that in the 5'-3' direction the oligonucleotide remains: tail+Spacer+primer.

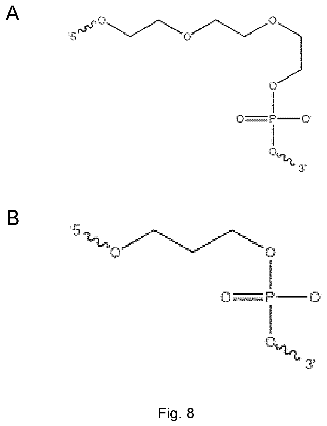

Spacer 9 (iSp9) is a triethylene glycol (Fig. 8A) spacer that can be incorporated into the 5'- or 3'- end of an oligonucleotide or internally. Multiple inserts can be used to create a longer separation space.

On the other hand, Spacer C3 (iSpC3), Fig. 8B, can be incorporated internally or at the 5'-ends of an oligo. Multiple C3 spacers can be added.

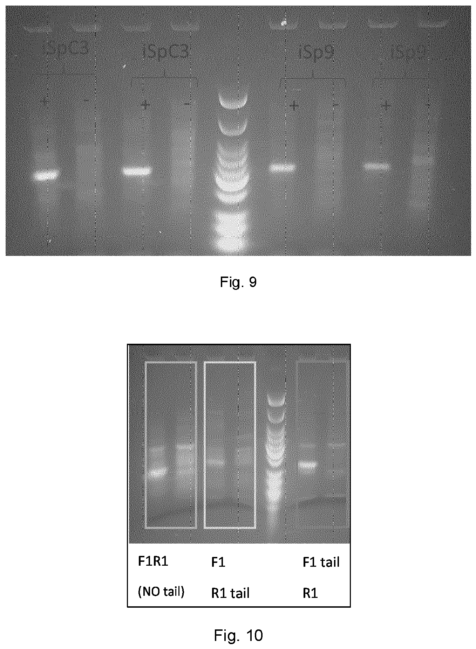

Using the same tail and the same pair of primers, an optimal amplification was demonstrated using both spacers (Fig. 9).

It was also observed that the tail could be added in both forward and reverse primers (Fig. 10).

Using spacer C3, the forward of the corresponding target was labelled with a tail.

### 3.2. Array for detection of antibiotic resistance genes

The capture probes were applied for the detection of antibiotic resistance genes in a lateral flow array design (Fig. 13). Each probe was assigned a target according to Table 3, and the corresponding target carried the complementary tail in one of the primers (table 4).

In Fig. 12 to 14, different results obtained by the array testing genomic DNA extracted from bacterial isolates can be observed. Demonstrating the correct detection of the 8 targets by their corresponding capture probe.

### Example 3: TLs use in singleplex and duplex for detection of SARS-CoV-2

### 1. Overview

The LF platform can be used for the detection of other targets simply by adding the corresponding tails to the specific primers designed, whether for singleplex, duplex or multiplex detection. Thus, apart from the bacterial resistance array, the capture probes and their corresponding tails have been applied in other assays for the detection of other amplicons.

In this case, it has been used for detection of SARS-CoV-2 in singleplex and duplex modes.

### 2. Materials and Methods

### 2.1. Materials en Equipment

RNAse inhibitor, Gel loading Dye and Ladder Quick Load Purple from New England BioLabs (M0307, B7025S and N0557S), TBE UltraPure^{™} and Agarosa UltraPure^{™} from Invitrogen (15581-044 and 16500-100), GelGreen^{®} Nucleic Acid Gel Stain from minipcrbio^{®} (RG-1550-01), PCR grade water, 10 mM TE and Sodium hydroxide. RT-RPA Commercial Amplification Kit. Primers and templates are described in the next section. SimpliAmp^{™} Thermal Cycler from Applied Biosystems (A24811).

For the LF platform, Materials and Methods are the same as reported in Example 1. In the case of SARS-CoV-2 detection, a singleplex and duplex LF platform, TL1 and TL2 were employed as test lines.

### 2.2. Primers and genome

The following genomes of virus from BEI resources were used: Quantitative PCR (qPCR) Control RNA from Heat-Inactivated SARS-Related Coronavirus 2, Isolate USA-WA1/2020, NR-52347, with a stock concentration of 1.35 × 10⁸cop/mL.

Primers previously reported for RPA amplification for the envelope (E) gene were used. Primers were obtained from Integrated DNA Technologies (IDT; Leuven, Belgium) and are summarized in Table 8. The oligos were resuspended in TE to a concentration of 100 µM and diluted as needed.

**Table 8. Sequences of the primers, tails and capture probes.**

| **Name** | **Sequence (5'-3')** |
|---|---|
| F E | Forward for SARS-CoV-2 detection |
| R E | Reverse for SARS-CoV-2 detection |
| TL1 | GTCGTGACTGGGAAAACTTTTTTTTTTTTTTT |
| TL2 | ACCACCACGACCAATTTTTTTTTTTTTTT |
| Tail 1 | GTT TTC CCA GTC ACG AC |
| Tail 2 | TTG GTC GTG GTG GTG GTT T |

### 2.3. RT-RPA

2 µL of the sample was used for the RT-RPA reaction. Briefly, each RT-RPA reaction was set to a final volume of 25 µL, 40 units of RNase Inhibitor were added, and the reaction was activated with 14 mM magnesium acetate. RT-RPA was then performed on the thermal cycler at 37 °C for 25 min.

### 3. Results

### 3.1. Singleplex detection

As shown in Fig. 20, different amounts of amplified E gene have been tested in the LF by using the probe (TL1) as test line.

### 3.2. Duplex detection

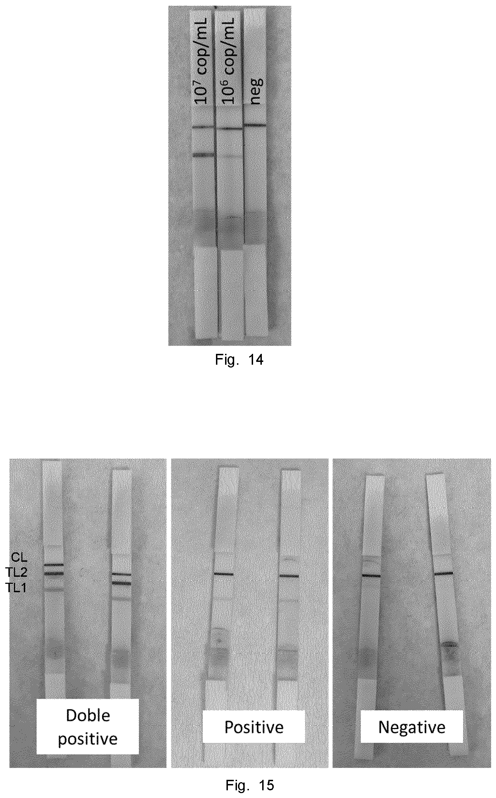

For a duplex detection of SARS-CoV-2, two different test lines (TL1 and TL2) were printed onto the nitrocellulose, and the corresponding tails were used to label de forward primer. As shown in Fig. 15, it was possible to obtain a double positive result or a single positive result depending on if the amplification product was obtained with one of the forwards labeled or both.

## Claims

1. A set of primer pairs for the multiplexed detection of a series of different target nucleic acids wherein the set contains at least two types of primer pairs being each type of primer pair specific for a different target nucleic acid wherein, for each given type of primer pair:
(i) The forward primer contains a target-specific region which is capable of specifically hybridizing to a target nucleic acid within the series of different target nucleic acids and a barcode region which comprises a sequence selected from the group consisting of SEQ ID NO: 1 to 8 or a variant thereof capable of specifically hybridizing to a nucleic acid having a sequence which are complementary to SEQ ID NO.1 to 8 and,
(ii) the reverse primer contains a target-specific region which is capable of specifically hybridizing to said target nucleic acid and which is labelled in its 5' end with a detectable tag
wherein the barcode region within the forward primer in a type of primer pairs is different from the barcode regions within the forward primers in the other types of primer pairs.

2. The primer set according to claim 1 wherein the target-specific region and the barcode region within the forward primer from each type of primer pair are connected by a spacer and/or wherein the target-specific region and the detectable tag in the reverse primer from each type of primer pair are connected by a spacer group.

3. The primer set according to claim 2 wherein the spacer group is a polyethyleneglycol group or alkyl group.

4. The primer set according to claim 3 wherein the spacer group has the following structure 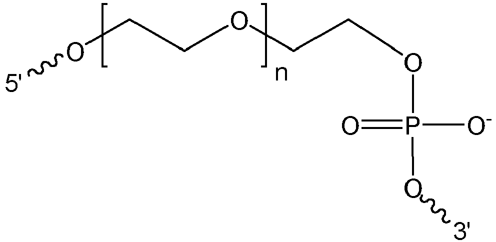 wherein n is from 0 to 18.

5. The primer set according to claim 4 wherein the spacer group has the structure

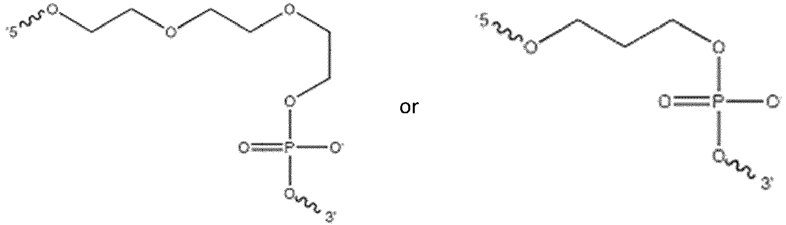

6. The primer set according to any of claims 1 to 5 wherein the length of the barcode region in the forward primers within each type of primer pair is of between 12 and 20 nucleotides.

7. The primer set according to any of claim 6 wherein the length of the barcode region in the forward primers within each type of primer pair is of 14 nucleotides.

8. A method for the multiplexed amplification of a series of target nucleic acids in a sample comprising contacting the sample with a set of primer pair as defined in any of claims 1 to 7 wherein the set of primers contains primer pairs containing forward and reverse primers specific for each of the target nucleic acids in the sample, wherein said contacting is carried out under conditions adequate for the amplification of the target nucleic acid thereby generating amplicons from each target nucleic acid, wherein each amplicon contains a tail comprising the sequence of the barcode region forming part of the specific forward primer and the detectable tag forming part of the specific reverse primer.

9. The method according to claim 8 wherein the amplification is an isothermal amplification.

10. A method for the multiplexed detection of a series of target nucleic acids in a sample comprising the steps of:
(iii) Generating amplicons from each type of target nucleic acid in a sample by means of a method as defined in claims 8 or 9,
(iv) Contacting the amplicons formed in step (i) with a support, said support containing different types of capture probes immobilized each of them at spatially predefined positions within the support, wherein each type of capture probe contains a region which contains a sequence complementary to a type of barcode region present in the amplicons, said contacting being carried out under conditions adequate for the hybridization between the tail in each type of amplicon and the complementary barcode region in the capture probes
wherein detection of the detectable label at one or more of the spatially predefined positions is indicative that the sample contains the target nucleic acid which forms part of the tailed amplicon containing a sequence which is complementary to the sequence of the capture probe.

11. The method according to claim 10 wherein the capture probe contains 16 nucleotides of which 14 of them are complementary to the barcode region.

12. The method according to any of claims 10 or 11 wherein the method is carried out in a lateral flow device, the device comprising
(i) a sample loading region which contains a porous substrate in which the metal nanoparticles, latex particle, magnetic beads, carbon nanoparticle or quantum dots coupled with a ligand that specifically binds the detectable tag are adsorbed,
(ii) a test region in which the capture probes are immobilized at the spatially predefined positions,
(iii) a channel which defines a flow path between the sample loading region and the test region
wherein the sample is applied to the sample loading region under conditions adequate for the flow through the channels of the amplicons within the sample and of the metal nanoparticles, latex particle, magnetic beads, carbon nanoparticle or quantum dots coupled with a ligand that specifically binds the detectable tag into the test region and wherein the detection is carried out in the spatially predefined regions within the test region.

13. A set of probes comprising different types of probes, wherein each type of probe contains a region having a sequence substantially complementary to barcode region wherein the barcode regions are as defined in any of SEQ ID NO:1 to 8 or a variant thereof capable of specifically hybridizing to a nucleic acid having a sequence which are complementary to SEQ ID NO.1 to 8 wherein the region having a sequence complementary to a type of barcode region in each type of capture probe is different from that in the other capture probes of the set.

14. The set according to claim 13 wherein the capture probe contains 16 nucleotides of which 14 of them are complementary to the barcode region.

15. The set of probes according to any of claims 13 or 14 wherein the probes are immobilized on a support and wherein each type of probe is immobilized at a spatially predefined region in the support.
